# EUROPEAN PATENT APPLICATION

(11) **EP 2 019 137 A1**
(43) Date of publication of application: **28.01.2009**
(21) Application number: 07743215.1
(22) Date of filing: 11.05.2007
(51) Int. Cl.: C12N 15/09, A01H 1/00, A01H 5/00, C07K 14/415, C07K 16/16, C12Q 1/68, G01N 33/53

(54) **METHOD FOR PRODUCTION OF CLEISTOGAMOUS RICE AND METHOD FOR SELECTION OF CLEISTOGAMOUS RICE**

(30) Priority: 12.05.2006 JP 2006134357
(71) Applicant: Incorporated Administrative Agency National Agriculture and Food Research Organization, Ibaraki 305-8517 (JP)
(72) Inventor: YOSHIDA, Hitoshi, Niigata 943-0154 (JP); OHMORI, Shinnosuke, Niigata 943-0837 (JP); NAGATO, Yasuo, Tokyo 176-0004 (JP); ITOH, Junichi, Tokyo 114-0002 (JP); SATOH, Hikaru c/o Kyushu University National University Corporation, Fukuoka 812-8581 (JP)
(74) Representative: Graf von Stosch, Andreas
(86) International application number: PCT/JP2007/059780
(87) International publication number: WO 2007/132789

(57) **Abstract**

The present invention provides a polypeptide in which the amino acid at position 45 of an amino acid sequence represented by SEQ ID NO: 1 is substituted with a hydrophilic amino acid; or a polypeptide having deletion, insertion, substitution, or addition of one or several amino acids other than the amino acid at position 45 in the above amino acid sequence. This makes it possible to realize the production and selection of a cleistogamous plant which has a normal fertility and is suitable for practical use.

SEQUENCE LISTING

<110> Incorporated Administrative Agency National Agriculture and Food
      Research Organization

<120> Method for Production of Cleistogamous Rice and Method for
      Selection of Cleistogamous Rice

<130> AF0722

<140> PCT/JP2007/059780
<141> 2007-05-11

<150> JP 2006-134357
<151> 2006-05-12

<160> 23

<170> PatentIn Ver. 2.1

<210> 1
<211> 224
<212> PRT
<213> rice

<400> 1
Met Gly Arg Gly Lys Ile Glu Ile Lys Arg Ile Glu Asn Ala Thr Asn
  1               5                  10                  15

Arg Gln Val Thr Tyr Ser Lys Arg Arg Thr Gly Ile Met Lys Lys Ala
             20                  25                  30

Arg Glu Leu Thr Val Leu Cys Asp Ala Gln Val Ala Ile Ile Met Phe
         35                  40                  45

Ser Ser Thr Gly Lys Tyr His Glu Phe Cys Ser Pro Ser Thr Asp Ile
     50                  55                  60

Lys Gly Ile Phe Asp Arg Tyr Gln Gln Ala Ile Gly Thr Ser Leu Trp
 65                  70                  75                  80

Ile Glu Gln Tyr Glu Asn Met Gln Arg Thr Leu Ser His Leu Lys Asp
                 85                  90                  95

Ile Asn Arg Asn Leu Arg Thr Glu Ile Arg Gln Arg Met Gly Glu Asp
            100                 105                 110

Leu Asp Gly Leu Glu Phe Asp Glu Leu Arg Gly Leu Glu Gln Asn Val
        115                 120                 125

Asp Ala Ala Leu Lys Glu Val Arg His Arg Lys Tyr His Val Ile Thr
    130                 135                 140

Thr Gln Thr Glu Thr Tyr Lys Lys Lys Val Lys His Ser Tyr Glu Ala
145                 150                 155                 160

Tyr Glu Thr Leu Gln Gln Glu Leu Gly Leu Arg Glu Glu Pro Ala Phe
                165                 170                 175

Gly Phe Val Asp Asn Thr Gly Gly Gly Trp Asp Gly Gly Ala Gly Ala
            180                 185                 190

Gly Ala Ala Ala Asp Met Phe Ala Phe Arg Val Val Pro Ser Gln Pro
        195                 200                 205

Asn Leu His Gly Met Ala Tyr Gly Gly Asn His Asp Leu Arg Leu Gly
    210                 215                 220

<210> 2
<211> 1210
<212> DNA
<213> rice

<400> 2
ggagttggga atccgttgct gtcgtacgtc tcgtctatgg cgtgctgtat gtcctctcct 60
tctttcttcc tttccaaata agcactcctc ttcttcctcc tcctcctcct ccgcctcctc 120
ttctcattcc caactcatcg atccatcagt agctagctag ctagctagct agctgcattg 180
tccggcgaga gagatagctg ctgcaggggg cggccatggg gaggggcaag atcgagatca 240
agcggatcga gaacgcgacc aacaggcagg tgacctactc gaagcgccgc acggggatca 300
tgaagaaggc cagggagctc accgtgctct gcgacgccca ggtcgccatc atcatgttct 360
cctccaccgg caagtaccac gagttctgca gcccttccac cgacatcaag gggatctttg 420
accgctacca gcaagccatc ggcaccagcc tttggatcga gcagtatgag aatatgcagc 480
gcacgctgag ccatctcaag gacatcaacc gcaacctgcg caccgagatc aggcaaagga 540
tgggagaaga tctggacggg ctggagttcg acgagctgcg cggtcttgag caaaatgtcg 600
atgccgccct caaggaggtt cgccacagga agtatcatgt gatcaccaca cagactgaaa 660
cctacaagaa aaaggtgaag cactcctacg aggcgtacga gactctgcag caggagctgg 720
ggttgcgcga ggagccggcg ttcgggttcg tggacaacac cggcggcggg tgggacggcg 780
gcgccggcgc cggcgcggcg gcggacatgt tcgccttccg cgtggtgccc agccagccca 840
acctgcacgg catggcctac ggcggcaacc acgacctgcg cctcggttga tatatcatcg 900
ccgccgccgc cgccgccgcc ggcggcctgc tcgtcgccgg ccggccggcc gccggacacc 960
gtatcatcgc tcgatctaga taacaagccg acgaataaat tgcagtgctc gtgttactta 1020
cctgcatgcg catgcattat tatcgatcaa cgcaagaact tcatttcatt taccaaacca 1080
cttgatgtgt gctatgatcc agcggttaaa ccgtgtgttg tctcttgaca ttttgtacca 1140
gttactgatg atgttgtatt atgtatgcgt ggtgtgcaac tgtgcttgga taatttcaca 1200
cttatttgtc 1210

<210> 3
<211> 9965
<212> DNA
<213> rice

<400> 3
tcctgcctag tgtaaattaa ggcgcactgt ggcttggcta ctgctacttt tataaagttt 60
ttgcactatg ggatgggttg tttcttaggg agttgcttgg taatattgtg cacggcagta 120
gctagtttct tcttcaaggg tagtttggta taagattgta catgagttgt tcgggtaaca 180
atatagcaat atatataaga tgatttttac cattattaag gaggtaccac caggtaatta 240
aggtattgag tatagttacc attcaaatct aaccgttgaa ctattatgtc atgatattat 300
atatggtcat aactttggct tgatatatct tccctatcta gttttcgctc ggtctccatg 360
gcatccaata tctctaagag atttacgctg gcctctgtca cccgccccta tggtcgtcgt 420
gtctgtggtg ctagtaccgg gttggaggag atcaaaaggt catcgagccg gtccattgct 480
gcatccaccg cactaagctc atcgctatgg ccgctaggtg acagcggcga tacgtcgggg 540
aaggatgtta aggacgaatg cattggagga acatggagtg ggtggtgcga aaagaaaatt 600
aatcataacc tttatagggt gtcggaaggc ttggtacccg gtgggactag gcaccgacgc 660
tactagatac gaaatgattc tcagccattt gatgatgctc aatagacggc cacgatttgg 720
tctatgtctc ccctcaagga ttgtaaaaag ttctctatat gtattggaat ttttcttttc 780
tggagaattt tttacttgat gtagtaattg ctatgatact tggcatgaat caaggaatgc 840
aagctagaaa cctttgtgca tgtaagcatc gttgaccaaa atatatagcc caaaatttcc 900
aacaatatgt attcttttgg atcacatatg tatgcactcc taccttagtc aatggaagca 960
tatgaatgcc cattgagtat tggagtaaac ctacacgaaa gagaagagaa aaaaaaaagt 1020

atcgttgctg ctggtaataa aagaaaagga ggtactccta gatggaaaca ggtctcttgt 1080
cactcacccc tgtagtttgt gcaatgacaa gtcacataca aagtgctgga agtgatagta 1140
tgtgggcatt aagtggtgcc tggactagaa agttgcagcg agtgttttcc acttgtgtcc 1200
aatggcgcat gattgaccgt gtcgtcctcg tcgccgttgc atttatggaa ctgagctgcc 1260
catcccctct cctccgtagc aaaggcatca cagattcaca ggtcgcccat cacgtcgatc 1320
gatcagtaac tgttacagca agcatgtaga atagccattt ctgaacctgc gaggttgcat 1380
ctatccatgg ttaaaacaag tcgccgtact acgaaccatt ggtgccatat gtcctttgat 1440
tcgttggggg tgacatgact agtcacgagc tgcctatatg taacactaga agaggcttct 1500
agtggtcgta gtagcttggt ttggttgctg gacaatgaga gttggtagca agaggtgcac 1560
cctgctgtcc ccccggttca gggataaaat gaagcacagg ctggaactta gattgtctag 1620
agatggtaat tggtcaattg gtgaagttgt gacttgtgat tacgagcatc ttgctcttgc 1680
aggacattgc tctccccttc gctcgtaatt ggtagtttgt tgtcattggc cttgtttgga 1740
tcctccgggc tattaaatag ctatccggaa tcttgctatt taggattatt aaacgtagat 1800
taccgacaaa accgatccca taacccctag actattttgc gagacgaatc taatgatgta 1860
tattaatcca taattagcgg ctgattactg tagcatcact gtagcaaatc atggattaat 1920
atacctcgtt agattcgttt cgcaaaatag cctaggggtt atggaatgag ttttgtcagt 1980
attctaagtt taatactctt aaatagcaag atttcggagg gctatttaat agccctccgg 2040
atccaaacag ggccattgtc ttttaaaaac acctaggggt cttctggcca gctccacgag 2100
atggtgggtt agacggcctg ggttcaaaac ctcacccctt ctaattattt gatattatgt 2160
cattttctaa tattcgcgtc tgttttgttg tcatcgtctt tctaagcgtg cgtgcttgca 2220
aaggaaagga gtagagagag agagaggcac atcagcagat cgagagagag aagctggtat 2280
gacaaaagaa aagaaaagac agaagatgac tcatgcacac ttgcgaaatc aaaccaaaag 2340
agaatcaaag cgaggcaata tgtacgcgca gaagcaatga tgaagcgaaa gccgaaatgg 2400
tgctaaccgt tgggcggcca gccacgcatg cagcgaatcg gtcgatcgat catgcagctg 2460
ctaacacaca cacaagagaa gacgagacga aactcagccg gccggtgatt cgatcgagag 2520
taggtgacac agccaagcag gatagaggct attttagga gagcttctag ctgctgcaac 2580
ttctctcaga attagaagct cccccaaaca gtatatcttt tggttcatat tctaagaagt 2640
tgtagttgta gaatccagaa agtgaaccag aagctagaag ctgaaaaacc tagcttttca 2700
atattctcag aagctgtagt tgtaaaatct agaaaatgaa tcagaaacca aaaaaatgaa 2760
aaatccagct tttctagatt cttataaatt gtctacaaat cagctgcttc tcataatttt 2820
aaactccaca aatactgcag gatccgtctc cgcgaaccga gagacgttac ggtatacgga 2880
gtgggcacga ttgctttcac gagttgggaa tccgttgctg tcgtacgtct cgtctatggc 2940
gtgctgtatg tcctctcctt ctttcttcct ttccaaataa gcactcctct tcttcctcct 3000
cctcctcctc cgcctcctct tctcattccc aactcatcga tccatcagta gctagctagc 3060
tagctagcta gctgcattgt ccggcgagag agatagctgc tgcagggggc ggccatgggg 3120
aggggcaaga tcgagatcaa gcggatcgag aacgcgacca acaggcaggt gacctactcg 3180
aagcgccgca cggggatcat gaagaaggcc agggagctca ccgtgctctg cgacgcccag 3240
gtcgccatca tcatgttctc ctccaccggc aagtaccacg agttctgcag cccttccacc 3300
gagtctgtat ctccatctcc acttcgactc tatgatcttc tccatcctga ttatactata 3360
tatataactg catcatatcg atcttaattg catcctgctg cagcatcaag gggatctttg 3420
accgctacca gcaagccatc ggcaccagcc tttggatcga gcagtatgag gttgatttgt 3480
ttaatttctt cctttcttcc cctccgctag ctgcaaccaa gctgctcact tcgattctct 3540
ccttatgatt tgttttctca tcttttgctc gattcgtctt ctcaatttcc actccagttt 3600
ctttccttta attctatgtc ccaactctct tctgctcgtt aattggtttg gtttggtttg 3660
ttttcatcca tcagaatatg cagcgcacgc tgagccatct caaggacatc aaccgcaacc 3720
tgcgcaccga gatcaggcca gcacaaattt caccccttta attactttcc tctcttctcc 3780
ttaattaatc cctcttagta tatagttact gggggatttt agcttgaaca agagatccaa 3840
atttacaacg tttttcaaga tatgtactgc tggatctgaa tataaacaac catacgtgtg 3900
tgcaggcaaa ggatgggaga agatctggac gggctggagt tcgacgagct gcgcggtctt 3960
gagcaaaatg tcgatgccgc cctcaaggag gttcgccaca ggaaggcatg cacgcacgca 4020
cgccctaatt aaactacgaa ccctaatttg ctcaatacta aaacgtcgtt caattcctta 4080
attcaagggt ggtagatcct tgtattttgt gcacactaga tctacggttc atgatcagat 4140
ccgtgcatat ttcatgcttc tcgctacaga tctacgagtt tcttgaaccc ttcttggctt 4200
cttgcatgtg tggaaattat attgatggtt tttaatttgg cctcggttgc ttgctcgtat 4260
taaacaagat aattttaagg tttatgcttt atagaaacat aatttatgtg tactattgtt 4320
ttatcttaca actagtagga tgtatgctca ttgatctatt ggactgtaac atattaaact 4380
ttctgcttca tgtatcctag ccttttaatt tgtttcagtt ttctcatgat taattcggtt 4440
ttaatttctg cagtatcatg tgatcaccac acagactgaa acctacaaga aaaggtcag 4500
gaataagcca tgcccatttt ctcagtattt gtttctggtt aaatttatat atatatatat 4560
atatatatat atatatatat atatatatat atatatatat accaccaatt ttattccttc 4620
ctcttgaaac tgttggctat aggtgtacat ctactgtgga ttttattcct tcccttggaa 4680
gctgttgact atgtgtgcat ctcttgtgga tatataggcc ggatttaatc aatccattat 4740

cttaaaaaaa atactgccaa tattatgcct aataatgcag caatattagt atatatatgt 4800
atgttcttct ggtgcagtaa taagggtata tctagatatc tgcacaagcc tatataagtg 4860
tatatttatc ttccccaatt ccttacttta attgcttcac ttttgtggcc accatcactg 4920
cttaaatttg cttagtttgg ccaataagtt cgcacttgca gtgtcgtcta tgttcacatg 4980
tctttctgaa gagctatatg ctatcccttt gattgtacgt tgttcacatt tttcctgacg 5040
atattaagtt cctccgggta aaatatacat tgatcccgtg aactaaattg cactcaccca 5100
ataacctgca ttttgcctta ttgcgtgcta atttagccat atacatagcc atggatctcc 5160
cagtttctgt acttgaacag tcgaagctag ctaagctagc ccttgaatcg atcaaaagcc 5220
aaaataaaag atctgacaag caccatgacg cctctcaacc gaaacatgca tgctaatgta 5280
cagtgtgcat taccatgcgt gtctgtgcat ttccatacta tctacacatc aatatttgat 5340
cgaggctacc tgatctacac atctagtcta agctatacac atagccatca gtagatgtac 5400
atcgatgtta attggagcat gcattgtcat gtgcattcct atacattaca tgatgaacaa 5460
cgcacgcaat ataatgcgat ctgcgatgca gttttctgtc ttttttgcaa gctagaactc 5520
atgacaaaca gcaaaggaca gtagtatgaa ttgggcatgt atctgcatat atatgcgtat 5580
atgtatctcc tgttaagcaa acaactaagc caggatcggt agctatataa actagctgta 5640
cctagagacg atgaagcatg catgcttatt ttggtccccc ggctatttag gagagtagca 5700
tgtcaaacac ggatttttat gagagattgc atcgcggcgt gatacctttt ggtagataag 5760
ggacggcatt gaggctttat gatttgcaag aatttggaaa ccaaaccatt acggccgctg 5820
gcttactgaa tctttagtgg ggtgtactac taattaatta ctcagcttga acaaccgagc 5880
aaaagcgaat acttttgact ttacattcaa tcgactttaa tagggagtac tctttgttca 5940
ttgttttgta gttttacctg cggttagctg gtcagaatat acacacatat acatatatat 6000
atgtagctga ataagtaatt cattgcatcg aggggattgt taaccattca tgcatatcta 6060
actgtgttgt cggctgaggt gaaaattaac cagttcaagc atgcatgttg gcatatagta 6120
taagctagca catcacaaat tcacaatcac cttcaagtgt ttaatttcgc tgcattagct 6180
actcctttgg tcttgttacg tgagagagct gacagagagt agtgtataaa ctctgaacaa 6240
agaactgcac tttgatatgg agctagctag aagatgactt tgaatctgta tatatatctt 6300
tgtcattgtg atatgggaga ggataacttg cttaaatttt atgtgagtga ctgttcatag 6360
aaataaactt gacgattgtt tcttgtgtgc tatagcatgc tgtccgaaga tgcacacatg 6420
tatatataca cattgcagtg acactctact tttacttgca cacatatgct gggccctgtg 6480
tctatacatg catgcacatg atataatttg tctgtctata gctaatagag attggtttct 6540
acattaattg atccggtgtg tgcatgcata taatgcaggt gaagcactcc tacgaggcgt 6600
acgagactct gcagcaggag ctggtaacct atcaagcatt tttttttcag ttatagccag 6660
aaaattattt gttaattagt ttctccaatg caggctagct aatttgattg aactctactg 6720
ttggatgaat gtttgaatta attcaggggt tgcgcgagga gccggcgttc gggttcgtgg 6780
acaacaccgg cggcgggtgg gacggcggcg ccggcgccgg cgcggcggcg gacatgttcg 6840
ccttccgcgt ggtgcccagc cagcccaacc tgcacggcat ggcctacggc ggcaaccacg 6900
acctgcgcct cggttgatat atcatcgccg ccgccgccgc cgccgccggc ggcctgctcg 6960
tcgccggccg gccggccgcc ggacaccgta tcatcgctcg atctagataa caagccgacg 7020
aataaattgc agtgctcgtg ttacttacct gcatgcgcat gcattattat cgatcaacgc 7080
aagaacttca tttcatttac caaaccactt gatgtgtgct atgatccagc ggttaaaccg 7140
tgtgttgtct cttgacattt tgtaccagtt actgatgatg ttgtattatg tatgcgtggt 7200
gtgcaactgt gcttggataa tttcacactt atttgccacg cttgctttat tagctttgac 7260
tatcaagcta ctgatcagag agttacctag ctcaaaggat taattattat gtatacagct 7320
caaatacatt aatgggctac tggctgctgc tgtccgagta attatttaca cctgtggtat 7380
atcgcttagt ccgttttgtt gggattttgt tgttggtctt gtgtcatctt ggtgtgtttt 7440
gttgtttttt tctctttatt tgtgtgtttt ttctttggct ctttggggag gtttttacct 7500
cctatgttct cttcttaatg aaatggcgca caattctttt gcgattttga ggaaaaaaaa 7560
aagattacct ccgtggccaa tcagcaagct cgccctcgcc ggcgccggtg agggcgagga 7620
gatcgccgtt gttggctgtt gtttgggctg gacagcccaa caattactgc tcagtagccc 7680
actctgggtg aaagcccgct catcccgata gagttaactt ccgtgggccg atttgtcgcg 7740
caaccaaggt ggcccgctcc gcctccttcg ttgcacgtgg gcccatacca cggtgaaacc 7800
atgtgtgggc cttatttggg cttaactctg gcccattaat aatcgctgga cagcgtccat 7860
gctagctact gcatttcttt tttcgatttc agtaccacgg aaagctataa gcatcaccag 7920
aatttggacg aaaattctat ataaacatca ccttgaattt acacgaaaat ttcacatttt 7980
caatgtatat tcgtttaaaa atgtaatcac cgcaggttag tgcactacac agccaaggag 8040
ataattgctg taattaacaa tgttatcctc gactaaatta aggtattaaa ttttctgtac 8100
gtactttcag tcgcctgttc aacatggaaa ttaagaaatg caaagtagaa gagacaaatt 8160
aacagcggct tttggccgcc tacttttcaa gttcatgtgc taagcatatg gcattgctaa 8220
tcatggggtc gtattaagga atcataatca tcatggtgat catcatcatc acaaggtgat 8280
taattaaata ataaagattt aaaggcggcc ggcaccagct gagactaaga ctgggctctg 8340
ggctcaatca gacgcaccgg actttcagct aaaagggcct cgtcaaaggt gcttgggcct 8400
ctccaagata ataaaaaaaa agccctttaa attaaaagtg ttaatcactt gatctcgcac 8460

taatcttctt cttgtatggg gctctccact ttactaattt tctttcagtc aaattggcag 8520
taattaacta cttcagatta tatatgtatt attaaatacc taagttttag cagttgcata 8580
tggttagctc atgagagtac tgtgaacaca tataataaat attcagtttc tttttgcagc 8640
tataggtcaa tgtgactgat atgtatatat tggtggtagg gcccagatgt ctgggggaca 8700
aagttgcttg ctcatctctt gcattgcaca ggagcctacc atggctatgg atcaacctaa 8760
gcttgtcgtt acgaattggc aaccattgaa gttggtcgga tgctatcttt actagcccat 8820
atagtcaaca tatgatcatc agaagcaacc gtaataatat atatatgaat cgatcattgg 8880
gacagggaca ccttgatcca taccatccat cgattcgatc atatctctct cgagatcatg 8940
agatgattga aattaacatc atcttgattt ctcaggtgat gtttggatac atggactaaa 9000
tgtcacatcg gatgtttgac actaattaga agtattaaac atagactact aataaaacta 9060
attgcataaa tttgcgagat aattttttta agcctaatta atttataact agcacatgtt 9120
taatgtagca tcacatatac taatcatgga ttatttaggc taaatagatt cgtctcttga 9180
attagtctag agttatggaa taggtttttt tcgcgaacgc gttatggaat aggttttgac 9240
attagtctat atttaatact tttaattagt gtcaaacatc caatctggca ggatccaaac 9300
atccccttag tgtgtctgac gaaactgaac acactactgt ccggttgatc aaccacgcaa 9360
aatcttcaga ctgttaataa tttcttcgtg aaaaaaaaag gaggagagga gcagttggat 9420
tattgttcag agaagacgac gagatagatg acatgcatca tctatcacca aatttcagta 9480
agacgtgtgc atgcatcttc cttgcagatt aagcactgta ttagctggga tatataatta 9540
taattagaat tcgtcgctat catgattcat gcacaagatt ttgtgtggtg aatgaaatct 9600
tacaacagta attagagtct agaagctagg taggtaggta taggttaatt agggacgaat 9660
agatggttgg atttggaagg tttagtgtct cttactaata atttataatt atcttttgat 9720
ggttctttca aagcctagct agataccatt acctacatat cattgcacta ctaatgcacg 9780
ctcaatgctc ttcgaataga tgcgacaatt cgtttactgt gttaagcctc tacacatgat 9840
ccattcatgt aatttaggag taataattaa tctgtttgca ttgtgcgtag gatacggtct 9900
ataagggata aaatccagtc taattaatca tactcggtat gaacatttga agaattaatg 9960
gcagt 9965

<210> 4
<211> 224
<212> PRT
<213> rice

<400> 4
Met Gly Arg Gly Lys Ile Glu Ile Lys Arg Ile Glu Asn Ala Thr Asn
1               5                   10                  15

Arg Gln Val Thr Tyr Ser Lys Arg Arg Thr Gly Ile Met Lys Lys Ala
            20                  25                  30

Arg Glu Leu Thr Val Leu Cys Asp Ala Gln Val Ala Thr Ile Met Phe
        35                  40                  45

Ser Ser Thr Gly Lys Tyr His Glu Phe Cys Ser Pro Ser Thr Asp Ile
    50                  55                  60

Lys Gly Ile Phe Asp Arg Tyr Gln Gln Ala Ile Gly Thr Ser Leu Trp
65                  70                  75                  80

Ile Glu Gln Tyr Glu Asn Met Gln Arg Thr Leu Ser His Leu Lys Asp
                85                  90                  95

Ile Asn Arg Asn Leu Arg Thr Glu Ile Arg Gln Arg Met Gly Glu Asp
            100                 105                 110

Leu Asp Gly Leu Glu Phe Asp Glu Leu Arg Gly Leu Glu Gln Asn Val
        115                 120                 125

Asp Ala Ala Leu Lys Glu Val Arg His Arg Lys Tyr His Val Ile Thr
    130                 135                 140

Thr Gln Thr Glu Thr Tyr Lys Lys Lys Val Lys His Ser Tyr Glu Ala

145                 150                 155                 160

Tyr Glu Thr Leu Gln Gln Glu Leu Gly Leu Arg Glu Glu Pro Ala Phe
                165                 170                 175

Gly Phe Val Asp Asn Thr Gly Gly Gly Trp Asp Gly Gly Ala Gly Ala
            180                 185                 190

Gly Ala Ala Ala Asp Met Phe Ala Phe Arg Val Val Pro Ser Gln Pro
        195                 200                 205

Asn Leu His Gly Met Ala Tyr Gly Gly Asn His Asp Leu Arg Leu Gly
    210                 215                 220

<210> 5
<211> 1210
<212> DNA
<213> rice

<400> 5
ggagttggga atccgttgct gtcgtacgtc tcgtctatgg cgtgctgtat gtcctctcct 60
tctttcttcc tttccaaata agcactcctc ttcttcctcc tcctcctcct ccgcctcctc 120
ttctcattcc caactcatcg atccatcagt agctagctag ctagctagct agctgcattg 180
tccggcgaga gagatagctg ctgcaggggg cggccatggg gaggggcaag atcgagatca 240
agcggatcga gaacgcgacc aacaggcagg tgacctactc gaagcgccgc acggggatca 300
tgaagaaggc cagggagctc accgtgctct gcgacgccca ggtcgccacc atcatgttct 360
cctccaccgg caagtaccac gagttctgca gcccttccac cgacatcaag gggatctttg 420
accgctacca gcaagccatc ggcaccagcc tttggatcga gcagtatgag aatatgcagc 480
gcacgctgag ccatctcaag gacatcaacc gcaacctgcg caccgagatc aggcaaagga 540
tgggagaaga tctggacggg ctggagttcg acgagctgcg cggtcttgag caaaatgtcg 600
atgccgccct caaggaggtt cgccacagga agtatcatgt gatcaccaca cagactgaaa 660
cctacaagaa aaaggtgaag cactcctacg aggcgtacga gactctgcag caggagctgg 720
ggttgcgcga ggagccggcg ttcgggttcg tggacaacac cggcggcggg tgggacggcg 780
gcgccggcgc cggcgcggcg gcggacatgt tcgccttccg cgtggtgccc agccagccca 840
acctgcacgg catggcctac ggcggcaacc acgacctgcg cctcggttga tatatcatcg 900
ccgccgccgc cgccgccgcc ggcggcctgc tcgtcgccgg ccggccggcc gccggacacc 960
gtatcatcgc tcgatctaga taacaagccg acgaataaat tgcagtgctc gtgttactta 1020
cctgcatgcg catgcattat tatcgatcaa cgcaagaact tcatttcatt taccaaacca 1080
cttgatgtgt gctatgatcc agcggttaaa ccgtgtgttg tctcttgaca ttttgtacca 1140
gttactgatg atgttgtatt atgtatgcgt ggtgtgcaac tgtgcttgga taatttcaca 1200
cttatttgtc                                                        1210

<210> 6
<211> 209
<212> PRT
<213> rice

<400> 6
Met Gly Arg Gly Lys Ile Glu Ile Lys Arg Ile Glu Asn Ser Thr Asn
  1               5                  10                  15

Arg Gln Val Thr Phe Ser Lys Arg Arg Ser Gly Ile Leu Lys Lys Ala
             20                  25                  30

Arg Glu Ile Ser Val Leu Cys Asp Ala Glu Val Gly Val Val Ile Phe
         35                  40                  45

Ser Ser Ala Gly Lys Leu Tyr Asp Tyr Cys Ser Pro Lys Thr Ser Leu
    50                  55                  60

Ser Arg Ile Leu Glu Lys Tyr Gln Thr Asn Ser Gly Lys Ile Leu Trp
65                  70                  75                  80

Asp Glu Lys His Lys Ser Leu Ser Ala Glu Ile Asp Arg Ile Lys Lys
                85                  90                  95

Glu Asn Asp Asn Met Gln Ile Glu Leu Arg His Leu Lys Gly Glu Asp
            100                 105                 110

Leu Asn Ser Leu Gln Pro Lys Glu Leu Ile Met Ile Glu Glu Ala Leu
        115                 120                 125

Asp Asn Gly Ile Val Asn Val Asn Asp Lys Leu Met Asp His Trp Glu
    130                 135                 140

Arg His Val Arg Thr Asp Lys Met Leu Glu Asp Glu Asn Lys Leu Leu
145                 150                 155                 160

Ala Phe Lys Leu His Gln Gln Asp Ile Ala Leu Ser Gly Ser Met Arg
                165                 170                 175

Asp Leu Glu Leu Gly Tyr His Pro Asp Arg Asp Phe Ala Ala Gln Met
            180                 185                 190

Pro Ile Thr Phe Arg Val Gln Pro Ser His Pro Asn Leu Gln Glu Asn
        195                 200                 205

Asn

<210> 7
<211> 710
<212> DNA
<213> rice

<400> 7
ctgctaagct agctggagga aggaggagga ggaggaggag gcgggatggg gcgcgggaag 60
atcgagatca agaggatcga gaactccacc aaccgccagg tgaccttctc caagcgcagg 120
agcgggatcc tcaagaaggc ccgcgagatc agcgtcctgt gcgacgccga ggtcggcgtc 180
gtcatcttct ccagcgccgg caagctctac gactactgct cccccaagac ctcgctatca 240
agaatcttgg agaagtacca gaccaattcc ggaaagatac tgtgggatga gaagcacaag 300
agccttagcg cggagattga tcgaatcaag aaagagaacg ataatatgca gattgagctc 360
aggcacttga aaggtgaaga tctaaactct ctgcagccca aagagctcat catgattgag 420
gaggcacttg acaatgggat agtgaacgtg aatgataaac tgatggacca ctgggaaagg 480
cacgtgagaa ctgataagat gctggaagac gagaacaagc tgctggcttt taaactgcac 540
cagcaagata tagcgctgag cgggagcatg agggatcttg agcttgggta ccatccagac 600
agggactttg cggcccagat gccgatcacc ttccgcgtgc agcccagcca ccccaacctg 660
caggagaaca attaagctgc taggttgccc cgccacttcg atcagttatc 710

<210> 8
<211> 210
<212> PRT
<213> rice

<400> 8
Met Gly Arg Gly Lys Ile Glu Ile Lys Arg Ile Glu Asn Ser Thr Asn
1               5                   10                  15

Arg Gln Val Thr Phe Ser Lys Arg Arg Ser Gly Ile Leu Lys Lys Ala
            20                  25                  30

Arg Glu Ile Gly Val Leu Cys Asp Arg Glu Val Gly Val Val Ile Phe
        35                  40                  45

Ser Ser Ala Gly Lys Leu Ser Asp Tyr Cys Thr Pro Lys Thr Thr Leu
    50                  55                  60

Ser Arg Ile Leu Glu Lys Tyr Gln Thr Asn Ser Gly Lys Ile Leu Trp
65                  70                  75                  80

Asp Glu Lys His Lys Ser Leu Ser Ala Glu Ile Asp Arg Val Lys Lys
                85                  90                  95

Glu Asn Asp Asn Met Gln Ile Glu Leu Arg His Met Lys Gly Glu Asp
            100                 105                 110

Leu Asn Ser Leu Gln Pro Lys Glu Leu Ile Ala Ile Glu Glu Ala Leu
        115                 120                 125

Asn Asn Gly Gln Ala Asn Leu Arg Asp Lys Met Met Asp His Trp Arg
    130                 135                 140

Met His Lys Arg Asn Glu Lys Met Leu Glu Asp Glu His Lys Met Leu
145                 150                 155                 160

Ala Phe Arg Val His Gln Gln Glu Val Glu Leu Ser Gly Gly Ile Arg
                165                 170                 175

Glu Leu Glu Leu Gly Tyr His His Asp Asp Arg Asp Phe Ala Ala Ser
            180                 185                 190

Met Pro Phe Thr Phe Arg Val Gln Pro Ser His Pro Asn Leu Gln Gln
        195                 200                 205

Glu Lys
    210

<210> 9
<211> 978
<212> DNA
<213> rice

<400> 9
tccccacctt aaagaccgga agaacggcaa ctccccaacc ctcttgtttc ttcgagagcg 60
aggtgaagat ttgtttcttg gtttgtgttc ttggagtgga ggaggaggag gagttggata 120
tggggcgcgg caagatcgag atcaagagga tcgagaactc gacgaaccgg caggtgacgt 180
tctcgaagcg ccgcgccggg atactcaaga aggcccgcga gatcggcgtg ctctgcgacg 240
ccgaggtcgg cgtcgtcatc ttctccagcg ccggcaagct ctccgactac tgcacgccca 300
agaccacgct gtcaaggatc ttggagaagt accagaccaa ctccgggaag atactctggg 360
atgagaagca caagagcctc agcgcagaga tcgatcgtgt caagaaggag aacgacaaca 420
tgcagatcga gctcaggcat atgaaagggg aggatctgaa ctccctgcag cccaaggagc 480
tgatcgcgat cgaggaggcg ctcaacaacg gccaggccaa tctgcgggac aagatgatgg 540
accactggag gatgcataaa aggaatgaga agatgctgga ggacgagcac aagatgttgg 600
cttttagggt gcaccagcag gaggtcgagc tgagcggcgg cataagggag ctggagctcg 660
gctaccacca cgacgacagg gacttcgcgg cctcgatgcc gttcaccttc agggtgcagc 720
ccagccaccc caacctccag caggagaagt aggctgctga gcactgccaa tttggctgaa 780
cctcctgctt gctagctttg catttcctca tgcgctgcta gatgtatctg tggtttctct 840
catgcctgtc tgaatgaact cacactgatg agagggtgtc agttgttttg tgctcctccc 900

agcattagca ctcagaattc agaaatgact caatgtcagt tgcttttgtg cttctcccag 960
cattagcact cagaattc 978

<210> 10
<211> 232
<212> PRT
<213> barley

<400> 10
Met Gly Arg Gly Lys Ile Glu Ile Lys Arg Ile Glu Asn Ala Thr Asn
1               5                   10                  15

Arg Gln Val Thr Tyr Ser Lys Arg Arg Ser Gly Ile Met Lys Lys Ala
            20                  25                  30

Arg Glu Leu Thr Val Leu Cys Asp Ala Gln Val Ala Ile Ile Met Phe
        35                  40                  45

Ser Ser Thr Gly Lys Tyr His Glu Phe Cys Ser Thr Gly Thr Asp Ile
    50                  55                  60

Lys Gly Ile Phe Asp Arg Tyr Gln Gln Ala Ile Gly Thr Ser Leu Trp
65                  70                  75                  80

Ile Glu Gln Tyr Glu Asn Met Gln Arg Thr Leu Ser His Leu Lys Asp
                85                  90                  95

Ile Asn Arg Asn Leu Arg Thr Glu Ile Arg Gln Arg Met Gly Glu Asp
            100                 105                 110

Leu Asp Ala Leu Glu Phe Glu Glu Leu Arg Gly Leu Glu Gln Asn Val
        115                 120                 125

Asp Ala Ala Leu Lys Glu Val Arg Gln Arg Lys Tyr His Val Ile Thr
    130                 135                 140

Thr Gln Thr Glu Thr Tyr Lys Lys Lys Val Lys His Ser Gln Glu Ala
145                 150                 155                 160

Tyr Lys Asn Leu Gln Gln Glu Leu Gly Met Arg Glu Asp Pro Ala Tyr
                165                 170                 175

Gly Phe Val Asp Asn Pro Ala Ala Gly Gly Trp Asp Gly Val Ala Ala
            180                 185                 190

Val Ala Met Gly Gly Gly Ser Ala Ala Asp Met Tyr Ala Phe Arg Val
        195                 200                 205

Val Pro Ser Gln Pro Asn Leu His Gly Met Ala Tyr Gly Gly Ser His
    210                 215                 220

Asp Leu Arg His Leu Arg Leu Gly
225                 230


<210> 11
<211> 1166
<212> DNA
<213> barley

<400> 11

attcggcacg agtccatcca cccctccttt actaaaccag cagctggaga aggagcagca 60
gcagctagcc gatctccttc cctcgtccac tttcttctcc gcgcatcgct gcgccgcgcg 120
cgcgacctag ctagcaagct cgctcgctcg caggcggtgg ccgcccgtgc gggcgcgcgc 180
gcgattgcgg ggacggagga ggtggatcgg ccggcggaga tggggcgggg gaagatcgag 240
ataaagcgga tcgagaacgc caccaacagg caggtgacct actccaagcg gcggtccggg 300
atcatgaaga aggcgcggga gctcaccgtg ctctgcgacg cccaggtcgc catcatcatg 360
ttctcctcca ccggcaagta ccacgagttc tgcagcaccg gcaccgacat caaggggatc 420
tttgaccgct accagcaggc catcgggacc agcctgtgga tcgagcagta tgagaatatg 480
cagcgcacgc tgagccatct caaggacatc aacaggaacc tgcgcaccga gatcaggcaa 540
aggatgggtg aagatctgga cgcgctcgag ttcgaggagc tgcggggcct tgaacaaaat 600
gtcgatgccg ctctcaagga ggttcgccag aggaagtatc atgtgatcac cacacagact 660
gaaacctaca agaagaaggt gaagcactcc caggaggcat acaagaatct gcagcaggag 720
ctgggcatgc gcgaggaccc ggcgtacggg ttcgtggaca acccggcggc gggcgggtgg 780
gacggtgtgg cggcggtggc gatgggcggc ggctcggcgg ctgacatgta cgccttccgc 840
gtggtgccca gccagcccaa cctgcacggc atggcctacg gcggctccca cgacctgcgc 900
cacctgcgcc tcggataatc gatcacctcg atcgctcctg ctagcttatg tatcaagtga 960
tcgattaagt taccacaata aaaggaaaac attgtgcgtt tgtatttgtg atatgttgtg 1020
gtctcgtgca cacgatttag ttcaatgtgt aattaacatc gtagtgctcg actgtgtatt 1080
gtatgatctg ctatgacttt ggttgtgaac tagctacttg ctactactat catatatatt 1140
ttggtacctt gtgatgacca tttcat 1166

<210> 12
<211> 209
<212> PRT
<213> barley

<400> 12
Met Gly Arg Gly Lys Ile Glu Ile Lys Arg Ile Glu Asn Ser Ser Asn
1               5                   10                  15

Arg Gln Val Thr Phe Ala Lys Arg Arg Ala Gly Leu Val Lys Lys Ala
            20                  25                  30

Arg Glu Ile Gly Val Leu Cys Asp Ala Glu Val Gly Val Val Ile Phe
        35                  40                  45

Ser Ser Ala Gly Lys Leu Tyr Asp Phe Trp Thr Pro Lys Thr Thr Leu
    50                  55                  60

Pro Arg Ile Leu Glu Lys Tyr Gln Thr Asn Ser Gly Lys Ile Leu Trp
65                  70                  75                  80

Asp Glu Lys His Lys Ser Ile Ser Ala Glu Ile Asp Arg Val Lys Lys
                85                  90                  95

Glu Asn Asp Asn Met Gln Ile Glu Leu Arg His Met Lys Gly Glu Asp
            100                 105                 110

Val Asn Ser Leu Gln Pro Lys Glu Leu Ile Ala Ile Glu Glu Ala Leu
        115                 120                 125

Thr Asn Gly Gln Thr Ser Leu Arg Asp Lys Met Met Asp His Trp Lys
    130                 135                 140

Met His Arg Arg Asn Glu Lys Met Leu Glu Glu Glu His Lys Leu Leu
145                 150                 155                 160

Ala Leu Arg Met His Gln Gln Asp Val Glu Leu Ser Ser Gly Met Arg
                165                 170                 175

Glu Met Glu Leu Gly Tyr His Gln Gly Arg Asp Phe Thr Ser Gln Met

180                 185                 190

Pro Phe Thr Phe Arg Leu Gln Pro Ser His Pro Asn Leu Gln Glu Asp
        195                 200                 205

Lys

<210> 13
<211> 965
<212> DNA
<213> barley

<400> 13
cggcacgagc tccacagaga caagggagga gaggtgtcct ctgggtcttt tgggtgctcc 60
gctcctaggt cttgtgcctt cccggcgagt agcttgttga gatggggcgc ggcaagatcg 120
agatcaagag gatcgagaac tcgtccaacc gccaggtgac cttcgccaag cgccgagccg 180
ggctggtgaa gaaggcccgc gagatcggcg tgctctgcga cgccgaggtc ggcgtcgtca 240
tcttctccag cgccggcaag ctctacgact tctggacgcc caagaccacg ctgccgagga 300
tcttggagaa gtaccagacc aactccggca agatcctgtg ggacgagaag cacaagagca 360
tcagcgcgga gatcgacagg gtgaaaaagg agaacgacaa catgcagatc gagctcaggc 420
atatgaaagg cgaggatgtg aactccctgc agcccaagga gctgatcgcc atcgaggagg 480
cgctcaccaa cggccagacc agcctcaggg acaagatgat ggaccactgg aagatgcaca 540
ggaggaatga gaagatgctg gaggaggagc acaagctgct ggctctgagg atgcaccagc 600
aggacgtcga gctgagcagc ggcatgaggg agatggagct cgggtaccat cagggtaggg 660
atttcacttc ccagatgccg ttcaccttcc ggctgcagcc cagccacccc aacttgcagg 720
aagacaagta ggccgctaaa tcatgccatg gcctgtctga actggtgctc catctacctg 780
caggctgcaa ctgctagctt caatttcctt gggcaacctt aaatgtatct tccttttcat 840
gtctggcttg atgaacttac cagtgctagt tgtttcccaa cacataaatt ttaattaaga 900
cagttgatgc caagatgttc ctggtcttag gactggaaaa caaggaactt ggtatatcat 960
gcttc                                                             965

<210> 14
<211> 229
<212> PRT
<213> wheat

<400> 14
Met Gly Arg Gly Lys Ile Glu Ile Lys Arg Ile Glu Asn Ala Thr Asn
1               5                   10                  15

Arg Gln Val Thr Tyr Ser Lys Arg Arg Ser Gly Ile Met Lys Lys Ala
            20                  25                  30

Arg Glu Leu Thr Val Leu Cys Asp Ala Gln Val Ala Ile Ile Met Phe
        35                  40                  45

Ser Ser Thr Gly Lys Tyr His Glu Phe Cys Ser Thr Gly Thr Asp Ile
    50                  55                  60

Lys Gly Ile Phe Asp Arg Tyr Gln Gln Ala Ile Gly Thr Ser Leu Trp
65                  70                  75                  80

Ile Glu Gln Tyr Glu Asn Met Gln Arg Thr Leu Ser His Leu Lys Asp
                85                  90                  95

Ile Asn Arg Asn Leu Arg Thr Glu Ile Arg Gln Arg Met Gly Glu Asp
            100                 105                 110

Leu Asp Ala Leu Glu Phe Glu Glu Leu Arg Asp Leu Glu Gln Asn Val

        115                 120                 125

Asp Ala Ala Leu Lys Glu Val Arg Gln Arg Lys Tyr His Val Ile Thr
    130                 135                 140

Thr Gln Thr Glu Thr Tyr Lys Lys Lys Val Lys His Ser Gln Glu Ala
145                 150                 155                 160

Tyr Lys Asn Leu Gln Gln Glu Leu Gly Met Arg Glu Asp Pro Ala Tyr
                165                 170                 175

Gly Phe Val Asp Asn Pro Val Ala Gly Gly Trp Asp Gly Val Ala Ala
            180                 185                 190

Val Ala Met Gly Gly Gly Leu Ala Ala Asp Met Tyr Ala Phe Arg Val
        195                 200                 205

Val Pro Ser Gln Pro Asn Leu His Gly Met Ala Tyr Gly Gly Ser His
    210                 215                 220

Asp Leu Arg Leu Gly
225

<210> 15
<211> 959
<212> DNA
<213> wheat

<400> 15
ggcacgagat ggggcggggg aagattgaga taaagcggat cgagaacgcc accaacaggc 60
aggtgaccta ctccaagcgc cggtcgggga tcatgaagaa ggcgcgggag ctcaccgtgc 120
tctgcgacgc ccaggtcgcc atcatcatgt tctcctccac cggcaagtac cacgagttct 180
gcagcaccgg caccgacatc aaggggatct ttgaccgcta ccagcaggcc atcggaccca 240
gcctgtggat cgagcagtat gagaatatgc agcgcacgct gagccatctc aaggacatca 300
atcggaacct gcgcaccgag atcaggcaaa ggatgggtga agatctggac gcgctggagt 360
tcgaggagct gcgcgacctt gaacaaaatg tcgatgccgc tctcaaggag gtccgccaga 420
ggaagtatca tgtgatcacc acgcagactg aaacctacaa gaagaaggtg aagcactccc 480
aggaggcata caagaatctg cagcaggagc tgggcatgcg cgaggacccg gcgtacggat 540
tcgtggacaa cccggtcgcg ggcgggtggg acggcgtggc ggcggtggcg atgggcggcg 600
gcttggcggc ggacatgtac gccttccgcg tggtgcccag ccagcccaac ctgcacggca 660
tggcctacgg cggctcccac gacctgcgcc tcggctaatc gatcacggct attgctccta 720
ctagcttata tatcgagtga tcaagttacc acaataagaa aaaaaaatct gtgtgtttgt 780
atttgtgaaa tgctgtgatc gatgatgcct tatgtcggtc tcgtgcacat gattcagttc 840
aatgtgtaat taacgccgta gtgctcgact gtgtattgta ttgcatgatg tgctatgact 900
ttggttgtga gctacttggt gctactatca tagtatatat tttggtactt gtgatgatc 959

<210> 16
<211> 227
<212> PRT
<213> wheat

<400> 16
Met Gly Arg Gly Lys Ile Glu Ile Lys Arg Ile Glu Asn Ala Thr Asn
1               5                   10                  15

Arg Gln Val Thr Tyr Ser Lys Arg Arg Ser Gly Ile Met Lys Lys Ala
            20                  25                  30

Arg Glu Leu Thr Val Leu Cys Asp Ala Gln Val Ala Ile Ile Met Phe
        35                  40                  45

Ser Ser Thr Gly Lys Tyr His Glu Phe Cys Ser Thr Gly Thr Asp Ile
    50                  55                  60

Lys Gly Ile Phe Asp Arg Tyr Gln Gln Ala Ile Gly Thr Ser Leu Trp
65                  70                  75                  80

Ile Glu Gln Tyr Glu Asn Met Gln Arg Thr Leu Ser His Leu Lys Asp
                85                  90                  95

Ile Asn Arg Asn Leu Arg Thr Glu Ile Arg Met Gly Glu Asp Leu Asp
            100                 105                 110

Ala Leu Glu Phe Glu Glu Leu Arg Asp Leu Glu Gln Asn Val Asp Ala
        115                 120                 125

Ala Leu Lys Glu Val Arg Gln Arg Lys Tyr His Val Ile Thr Thr Gln
    130                 135                 140

Thr Glu Thr Tyr Lys Lys Lys Val Lys His Ser Gln Glu Ala Tyr Lys
145                 150                 155                 160

Asn Leu Gln Gln Glu Leu Gly Met Arg Glu Asp Pro Ala Tyr Gly Phe
                165                 170                 175

Val Asp Asn Pro Ala Ala Gly Gly Trp Asp Gly Val Ala Ala Val Ala
            180                 185                 190

Met Gly Gly Gly Ser Ala Ala Asp Met Tyr Ala Phe Arg Val Val Pro
        195                 200                 205

Ser Gln Pro Asn Leu His Gly Met Ala Tyr Gly Gly Ser His Asp Leu
    210                 215                 220

Arg Leu Gly
225


<210> 17
<211> 1083
<212> DNA
<213> wheat

<400> 17
ggcacgagat ctcccctcct tccccccata ctctttcttc tccacccgtc gctgcgtcgg 60
ccgacctagc tagccagctc gctcgctcgc ggtggcgcgc gcgattgcgg ggtcggagga 120
ggtggatcgg gcggcggaga tggggcgggg gaagatcgag ataaagcgga tcgagaacgc 180
cacaaacagg caggtgacct actccaagcg ccggtcgggg atcatgaaga aggcgcggga 240
gctcaccgtg ctctgcgacg cccaggtcgc catcatcatg ttctcctcca ccggcaagta 300
ccacgagttc tgcagcaccg gcaccgacat caaggggatc tttgaccgct accagcaggc 360
catcgggacc agcctgtgga tcgagcagta tgagaatatg cagcgcacgc tgagccatct 420
caaggacatc aatcggaacc tgcgcaccga gatcaggatg ggtgaagatc tggacgcgct 480
ggagttcgag gagctgcgcg accttgagca aaatgtcgat gccgctctca aggaggttcg 540
ccagaggaag tatcatgtga tcaccacgca gactgaaacc tacaagaaga aggtgaagca 600
ctcccaggag gcatacaaga atctgcagca ggagctgggt atgcgcgagg acccggcgta 660
cgggttcgtg gacaacccgg ctgcgggcgg gtgggacggc gtggcagcgg tggcgatggg 720
cggcggctcg gcggcggaca tgtacgcctt ccgcgtggtg cccagccagc ccaacctgca 780
cggcatggcc tacggcggct cccacgacct gcgcctcggc taatcgatca cttcgatcgc 840
tcctactagc ttatatatca agtgatcgat caagttacca caataagaaa aaaattctgt 900
gtgtttgtat ttgtgaaatg ctgtgatcga tgatgcctta tctcggtctc gtgcacatga 960
gtcagttcaa tgtgtaatta acgccgtagt gctcgactgt gtattgtatt gtatgatctg 1020
ctatgacttt ggttgtgagc tactttgcca gtactataat atattttggt actttgtgat 1080

gtc                                                                 1083

<210> 18
<211> 208
<212> PRT
<213> wheat

<400> 18
Met Gly Arg Gly Lys Ile Glu Ile Lys Arg Ile Glu Asn Ser Ser Asn
1               5                   10                  15

Arg Gln Val Thr Phe Ala Lys Arg Arg Ala Gly Leu Val Lys Lys Ala
            20                  25                  30

Arg Glu Ile Gly Val Leu Cys Asp Ala Glu Val Gly Val Val Ile Phe
        35                  40                  45

Ser Ser Ala Gly Lys Leu Tyr Asp Phe Trp Thr Pro Glu Thr Thr Leu
    50                  55                  60

Pro Arg Ile Leu Glu Lys Tyr Gln Thr Asn Ser Gly Lys Ile Leu Trp
65                  70                  75                  80

Asp Glu Lys His Lys Ser Ile Ser Ala Glu Ile Asp Arg Val Lys Lys
                85                  90                  95

Glu Asn Asp Asn Met Gln Ile Glu Leu Arg His Met Lys Gly Glu Asp
            100                 105                 110

Val Asn Ser Leu Gln Pro Lys Glu Leu Ile Ala Ile Glu Glu Ala Leu
        115                 120                 125

Thr Asn Gly Gln Thr Asn Leu Arg Asp Lys Met Met Asp His Trp Lys
    130                 135                 140

Met His Arg Arg Asn Glu Lys Met Leu Glu Glu Glu His Lys Leu Leu
145                 150                 155                 160

Ala Leu Arg Met His Gln Gln Asp Asp Leu Ser Ser Gly Met Arg Glu
                165                 170                 175

Met Glu Leu Gly Tyr His Gln Gly Arg Asp Phe Thr Ser Gln Met Pro
            180                 185                 190

Phe Thr Phe Arg Leu Gln Pro Ser His Pro Asn Leu Gln Glu Asp Lys
        195                 200                 205

<210> 19
<211> 928
<212> DNA
<213> wheat

<400> 19
gaggagaggg aggagtcctc tgggtctggg tcttgggtgc tcctgggtcc tctttctttc 60
ttcccggcga gcgagcagct tgttgagatg gggcgcggga agatcgagat caagaggatc 120
gagaactcgt ccaaccgcca ggtgaccttc gccaagcgcc gggccgggct ggtgaagaag 180
gcccgcgaga tcggcgtgct ctgcgacgcc gaggtcggcg tcgtcatctt ctccagcgcc 240

ggcaagctct acgacttctg gacgcccgag accacgctgc cgaggatctt ggagaagtac 300
cagaccaact ccggcaagat cctctgggat gagaagcaca agagcatcag cgccgagatc 360
gacagggtga agaaggagaa cgacaacatg cagatcgagc tcaggcatat gaaaggcgag 420
gatgtgaact ccctgcagcc caaggagctg atcgccatcg aggaggcgct caccaacggc 480
cagaccaacc tcagggacaa gatgatggac cactggaaga tgcacaggag gaatgagaag 540
atgctggagg aggagcacaa gctgctggct ttgaggatgc accagcagga cgacctgagc 600
agcggcatga gggagatgga gctcgggtac catcagggta gggatttcac ttcccagatg 660
ccgttcacct tccggctgca gcccagccac cccaacttgc aggaagacaa gtaggccatc 720
gaatcctgcc gtggcctgtg tgaactgaag ctcctctacc tgcaggccac aactgctagc 780
ttcaatttcc gtgtgcaacc ttaaatgtat cttccttttc atgtctggcc tgatgaactt 840
aacaccgcta gttgcttccc aacacataac ttttaactaa gacagttcct gtccctgtca 900
agatgcttgt ataattgctg cctatcct 928

<210> 20
<211> 209
<212> PRT
<213> wheat

<400> 20
Met Gly Arg Gly Lys Ile Glu Ile Lys Arg Ile Glu Asn Gln Ser Asn
1               5                   10                  15

Arg Gln Val Thr Phe Ser Lys Arg Lys Asn Gly Ile Leu Lys Lys Ala
            20                  25                  30

Lys Glu Ile Ser Val Leu Cys Asp Ala Glu Val Gly Val Val Val Phe
        35                  40                  45

Ser Ser Ala Gly Lys Leu Tyr Asp Phe Cys Ser Pro Lys Thr Ser Leu
    50                  55                  60

Ser Arg Ile Leu Glu Lys Tyr Gln Thr Asn Ser Gly Lys Ile Leu Trp
65                  70                  75                  80

Asp Glu Lys His Lys Ser Leu Ser Ala Glu Ile Asp Arg Ile Lys Lys
                85                  90                  95

Glu Asn Asp Asn Met Gln Ile Glu Leu Arg His Leu Lys Gly Glu Asp
            100                 105                 110

Leu Asn Ser Leu Gln Pro Lys Glu Leu Ile Met Ile Glu Glu Ala Leu
        115                 120                 125

Asp Asn Gly Leu Thr Ser Leu His Glu Lys Gln Met Glu His Tyr Asp
    130                 135                 140

Arg Leu Val Lys His Gly Lys Met Leu Glu Asp Glu Asn Lys Leu Leu
145                 150                 155                 160

Ala Phe Lys Val His Gln His Asp Ile Ala Leu Ser Gly Ser Met Arg
                165                 170                 175

Asp Leu Glu Leu Gly Tyr His Pro Asp Arg Asp Phe Ala Ala Gln Met
            180                 185                 190

Pro Ile Thr Phe Arg Val Gln Pro Ser His Pro Asn Leu Gln Glu Asp
        195                 200                 205

Ser

<210> 21
<211> 1025
<212> DNA
<213> wheat

<400> 21
cgcccgcacc ggcccccaga gcgccagaag aaacgagccc ttcctggcgc cgccggagga 60
ggaagaagga ggagggatgg ggcgcgggaa gatcgagatc aagaggatcg agaaccagag 120
caaccgccag gtgaccttct ccaagcgcaa gaacgggatc ctcaagaagg ccaaggagat 180
cagcgtgctc tgcgacgccg aggtcggcgt cgtcgtcttc tccagcgccg gcaagctcta 240
cgacttctgc tcccccaaga cctcgctatc aagaatcttg gagaagtacc agaccaactc 300
cgggaagata ctgtgggatg agaaacacaa gagcctcagt gcggagattg atcgaatcaa 360
gaaagagaat gataacatgc agatcgagct caggcacttg aaaggcgaag atctgaactc 420
gctgcagccc aaagagctga tcatgattga ggaagcactt gataatgggc tgacaagtct 480
gcatgaaaaa cagatggagc actatgacag gctcgtgaaa catggtaaga tgctggaaga 540
tgagaacaag ctgctggcct ttaaagtgca ccagcatgat atcgccttga gcgggagcat 600
gagggatctt gagcttgggt accatccaga cagggacttt gcagcccaga tgccgatcac 660
cttccgtgtg cagcctagcc accccaatct gcaggaggac agctaggccg ccgactctcc 720
acgccactcc atcaaatgta tcccccactg gcttgtatgt ccatcatgca tcttgccagt 780
tgtatccttc gtttgttatc tgtttggacg aacaattgaa catgttatac gtagtagctt 840
gatgatgtgt cgatacagtc gggctctaaa agctgtgcag ccgccgcctg ccgcctgctg 900
atctgccata ctgtaagttt gcacgaccat tggagctgaa atatcagtgg actggtattg 960
tatctgcatg ttgtttccct gatcaccaca attgctggac ggtgaatagt ttgtaatgac 1020
aacag 1025

<210> 22
<211> 38
<212> DNA
<213> Artificial Sequence

<220>
<223> Description of Artificial Sequence: Artificially
      Synthesized DNA Sequence

<400> 22
cagggagctc accgtgctct gcgacgccca ggtcgcca 38

<210> 23
<211> 41
<212> DNA
<213> Artificial Sequence

<220>
<223> Description of Artificial Sequence: Artificially
      Synthesized DNA Sequence

<400> 23
tgcagaactc gtggtacttg ccggtggagg agaacaccat g 41

## Description

### TECHNICAL FIELD

The present invention relates to a method for producing a mother plant practically useful for production of genetically modified rice. In particular, the present invention relates to a protein which gives cleistogamy to a plant, a gene encoding the protein, and use of the protein and the gene.

### BACKGROUND ART

Commercialization of genetically modified crops has been demanded because of their great potentiality. However, the commercialization of genetically modified crops has been difficult since producers and consumers have strong concerns about the gene flow caused by crossing with the related wild species or the neighboring cultivars for the reasons that effect of transgenes on an environment for long period and others is not evaluated. Until effect of transgenes on an environment for long period and others is evaluated, preventive measures against the gene flow have been required for safety ensuring, from the view point of precautionary principle.

Generally, rice is a plant that performs self-pollination at a high frequency, but can perform cross-pollination at a low frequency since rice releases its pollen grains to the outside after its flowering. In outside cultivation, the ensuring of a distance between the genetically modified crops and the surrounding plants or the adjustment of a cultivation time of genetically modified crops (e.g. shift of their flowering stage) has been required to prevent genetically modified crops from being crossed with the surrounding wild plants or the related crops due to the release of pollen grains of the genetically modified crops. If these approaches are impossible, flower picking, removal of stamens, bagging of flowers, installation of wind-breaking nets, etc. are performed. However, they require enormous efforts, and the development of techniques for preventing the release of pollen grains of rice has been demanded.

Techniques for preventing the gene flow caused by the release of pollen grains of the genetically modified plants have been proposed such as maternal inheritance by chloroplast transformation, apomixis, and male sterility. However, chloroplast transformation and apomixes of rice have not been practically used. In addition, male-sterile rice exists but is not practically used because it requires artificial pollination for harvesting of its seeds.

As another technique for preventing the release of pollen grains, cleistogamy is known. As to barley, genetic analysis using cleistogamous cultivars has been made (see Patent Document 1 and Non-Patent Document 1, for example). It is revealed that cleistogamous cultivars exist according to the study on barley, and cleistogamy is controlled by small number of genes according to the genetic analysis.

Some reports on cleistogamous rice have been made so far (see Non-Patent Documents 2 and 3). Further, as rice having a trait similar to cleistogamy, "sheathed-panicle rice" has been reported (see Non-Patent Document 4). Some other reports have been made on non-flower-opening rice caused by morphological change of floral organs (see Non-Patent Documents 5 and 6).
[Patent Document 1]
   Japanese Unexamined Patent Publication No. 2005-229854 (published on September 2, 2005)
[Non-Patent Document 1]
   Theor Appl Genet 109, 480-7(2004)
[Non-Patent Document 2]
   J Fac Agric Hokkaido Univ. 53, 72-130(1963)
[Non-Patent Document 3]
   Current Science 50, 419-420(1981)
[Non-Patent Document 4]
   Rice Genetics Newsletter 3, 62-63(1986)
[Non-Patent Document 5]
   Development 130, 705-718 (2003)
[Non-Patent Document 6]
   Plant Cell Physiol.43, 130-135 (2002)

### DISCLOSURE OF INVENTION

As cleistogamous rice that has been reported so far, 'Heieidaikoku (d7)' mutant is presumed to cause other growth disturbances (e.g. dwarfism and short grain) due to pleiotropic expressions of the causal gene. Meanwhile, 'Dhundhuni' is presumed to show a complex inheritance pattern for cleistogamy (there are many genes associated with cleistogamy, and maternal inheritance is also involved in cleistogamy). Thus, both the 'Heieidaikoku (d7)' mutant and the 'Dhundhuni' are difficult to be used as mother plants for crossing.

"Sheathed-panicle rice", of which panicle is surrounded by sheath of flag leaf, is presumed to incur the risk of diseases after grain ripening and complicated harvesting. Therefore, "sheathed-panicle rice" is not suitable for practical use.

As rice exhibiting cleistogamy caused by morphological change of floral organ "lodicule" (corresponding to petal of dicotyledon) that functions as a locomotive of flowering, superwoman1 (spw1) mutant, Act::OsMADS3 transgenic plants, and others have been reported. However, they have morphologically changed stamens and severe sterility and are not suitable for practical use.

Thus, the development of cultivars having cleistogamy capable of preventing the release of pollen grains has been demanded. However, there exist no cleistogamous cultivars of rice for practical use in Japan. In addition, the existence of a gene giving cleistogamy and the mechanism to control cleistogamy were not revealed.

The present invention has been attained in view of the above problems, and an object of the present invention is to provide a technique for producing and selecting a practical cleistogamous plant that has normal fertility but does not affect agronomic traits such as plant type, and heading.

The inventors of the present invention obtained the sole cleistogamous rice mutant, superwoman1-cleistogamy (spw1-cls), from a mutant population mutagenized with chemical mutagen N-methyl-N-nitrosourea (MNU) by using *japonica* rice cultivar 'Taichung 65' as original cultivar. Specifically, the inventors of the present invention (1) selected from the above population mutant lines in which stamens were not exserted out of glume at flowering stage, and (2) selected a line that was normally fertile from the selected lines. Further, by map-based cloning of rice spw1-cls mutant, the inventors verified that cleistogamy was caused by mutation of a gene encoding SUPERWOMAN (SPW1) that is a class-B MADS-box protein, thereby accomplishing the present invention.

That is, a polypeptide of the present invention is a polypeptide which gives cleistogamy to a plant, wherein the polypeptide is: (A) a polypeptide comprising an amino acid sequence having substitution of an amino acid at position 45 in the amino acid sequence of a class-B MADS-box protein with a hydrophilic amino acid; or (B) a polypeptide comprising an amino acid sequence having deletion, insertion, substitution, or addition of one or several amino acids other than the amino acid at position 45 in an amino acid sequence of the polypeptide (A).

A polypeptide of the present invention is capable of giving cleistogamy to a plant. Considering the fact that the superwoman1 (spw1) mutant has morphologically changed stamen and severe sterility (see Non-Patent Document 5), it could not be expected at all that substitution of one amino acid in SPW1 gave cleistogamy to a plant.

A polypeptide of the present invention may be a polypeptide which gives cleistogamy to a plant, wherein the polypeptide is: (A) a polypeptide comprising an amino acid sequence having substitution of an amino acid at position 45 in the amino acid sequence of a class-B MADS-box protein of monocotyledon with a hydrophilic amino acid; or (B) a polypeptide comprising an amino acid sequence having deletion, insertion, substitution, or addition of one or several amino acids other than the amino acid at position 45 in an amino acid sequence of the polypeptide (A).

A polypeptide of the present invention may be a polypeptide which gives cleistogamy to a plant, wherein the polypeptide is: (A) a polypeptide comprising an amino acid sequence having substitution of an amino acid at position 45 in an amino acid sequence represented by SEQ ID NO: 1, 6, 8, 10, 12, 14, 16, 18 or 20 with a hydrophilic amino acid; or (B) a polypeptide comprising an amino acid sequence having deletion, insertion, substitution, or addition of one or several amino acids other than the amino acid at position 45 in the amino acid sequence of the polypeptide (A).

A polypeptide of the present invention may be a polypeptide which gives cleistogamy to a plant, wherein the polypeptide is: (A) a polypeptide comprising an amino acid sequence having substitution of an amino acid at position 45 in an amino acid sequence represented by SEQ ID NO: 1, 6, or 8 with a hydrophilic amino acid; or (B) a polypeptide comprising an amino acid sequence having deletion, insertion, substitution, or addition of one or several amino acids other than the amino acid at position 45 in the amino acid sequence of the polypeptide (A).

A polypeptide of the present invention is preferably such that the hydrophilic amino acid is threonine.

A polynucleotide of the present invention encodes the above polypeptide.

A polynucleotide of the present invention may be a polynucleotide that has a base sequence represented by SEQ ID NO: 5, or a polynucleotide that is capable of hybridizing under stringent conditions with the polynucleotide having a base sequence represented by SEQ ID NO: 5 and gives cleistogamy to a plant.

A plant of the present invention expresses the above polypeptide.

A plant of the present invention expresses the above polynucleotide.

A plant producing method of the present invention is a method for producing a plant to which cleistogamy is given, comprising any one of:
(I) the step of inducing mutation in a gene that encodes the class-B MADS-box protein, wherein the mutation corresponds to an amino acid substitution at position 45 of a class-B MADS-box protein;
(II) the step of introducing into a plant a gene being capable of inducing mutation in a gene that encodes a class-B MADS-box protein, wherein the mutation corresponds to an amino acid substitution at position 45 of the class-B MADS-box protein; and
(III) the step of suppressing expression of genes encoding class-B MADS-box protein other than SPW1 (polypeptide having an amino acid sequence represented by SEQ ID NO: 1).

A plant production kit of the present invention is a kit for producing a plant to which cleistogamy is given, wherein the kit comprises the above polynucleotide.

An oligonucleotide of the present invention is a fragment of the above polynucleotide, or an oligonucleotide comprising a complementary sequence of the base sequence of the fragment.

An oligonucleotide of the present invention may be an oligonucleotide that has a base sequence represented by SEQ ID NO: 23.

An oligonucleotide set of the present invention comprises a set of oligonucleotides including a fragment of the above polynucleotide, or an oligonucleotide comprising a complementary sequence of the base sequence of the fragment.

An oligonucleotide set of the present invention comprises an oligonucleotide that has a base sequence represented by SEQ ID NO: 22 and oligonucleotide that has a base sequence represented by SEQ ID NO: 23.

A screening method of the present invention is a method for screening a plant to which cleistogamy is given, comprising the step of hybridizing the above oligonucleotide with cDNA derived from a target plant.

A screening method of the present invention is a method for screening a plant to which cleistogamy is given, comprising the step of hybridizing an oligonucleotide having a base sequence represented by SEQ ID NO: 23 with cDNA derived from a target plant.

A screening method of the present invention preferably further comprises the step of hybridizing an oligonucleotide having a base sequence represented by SEQ ID NO: 22 with cDNA derived from a target plant.

A screening kit of the present invention is a kit for screening a plant to which cleistogamy is given, wherein the kit comprises the above oligonucleotide.

A screening kit of the present invention is a kit for screening a plant to which cleistogamy is given, wherein the kit comprises an oligonucleotide having a base sequence represented by SEQ ID NO: 23.

A screening kit of the present invention preferably further comprises an oligonucleotide having a base sequence represented by SEQ ID NO: 22.

An antibody of the present invention is capable of binding specifically to the above polypeptide.

A screening method of the present invention is a method for screening a plant to which cleistogamy is given, comprising the step of incubating the above antibody with a tissue extract derived from a target plant.

A screening kit of the present invention is a kit for screening a plant to which cleistogamy is given, wherein the kit comprises the above antibody.

Additional objects, features, and strengths of the present invention will be made clear by the description below. Further, the advantages of the present invention will be evident from the following explanation in reference to the drawings.

### BRIEF DESCRIPTION OF DRAWINGS

Fig. 1 is a view showing phenotypes of cleistogamous mutant spw1-cls.
Fig. 2 is a view showing comparison in plant type, panicle, and rice grain between cleistogamous mutant spw1-cls and a wild type.
Fig. 3 is a view showing structural change of floral organs in cleistogamous mutant spw1-cls and a causal gene of spw1-cls mutation.
Fig. 4 is a view showing function complementation of cleistogamous mutantspw1-cls by a wild-type SPW gene.
Fig. 5 is a view showing the analysis result of influences on OsMADS2 and OsMADS4 in terms of cleistogamy.

### BEST MODE FOR CARRYING OUT THE INVENTION

### [1: Polypeptide and polynucleotide]

### [1-1] Polypeptide

The present invention provides a polypeptide which gives cleistogamy to a plant. A polypeptide of the present invention is a polypeptide comprising an amino acid sequence having substitution of an amino acid at position 45 with a hydrophilic amino acid in the amino acid sequence of class-B MADS-box protein in a plant, or its mutant. The plant is preferably a monocotyledon, more preferably a *Poaceae* plant (e.g. rice, barley, wheat, rye, oat, proso millet, foxtail millet, or corn), most preferably rice, barley, or wheat.

Class-B MADS-box proteins in plants are classified into AP3 proteins and PI proteins. The AP3 proteins are SPW 1 of rice (SEQ ID NO: 1), HvAP3 of barley (SEQ ID NO: 10), TaMADS#51 of wheat (SEQ ID NO: 14), and TaMADS#82 of wheat (SEQ ID NO: 16). The PI proteins are OsMADS2 of rice (SEQ ID NO: 6), OsMADS4 of rice (SEQ ID NO: 8), HvPI of barley (SEQ ID NO: 12), WPI1 of wheat (SEQ ID NO: 18), and WPI2 of wheat (SEQ ID NO: 20). However, the class-B MADS-box proteins in plants are not limited to these proteins. Note that cDNA sequences of these proteins are as follows: SEQ ID NO: 2 (SPW of rice), SEQ ID NO: 11 (HvAP3 of barley), SEQ ID NO: 15 (TaMADS#51 of wheat), SEQ ID NO: 17 (TaMADS#82 of wheat), SEQ ID NO: 7 (OsMADS2 of rice), SEQ ID NO: 9 (OsMADS4 of rice), SEQ ID NO: 13 (HvPI of barley), SEQ ID NO: 19 (WPI1 of wheat), and SEQ ID NO: 21 (WPI2 of wheat).

A polypeptide of the present invention comprises an amino acid sequence having substitution of an amino acid at position 45 in the amino acid sequence of such a class-B MADS-box protein with a hydrophilic amino acid. As to the plant produced by the inventors of the present invention, the amino acid at position 45, isoleucine, in SUPERWOMAN1 (SPW1) protein, which is a class-B MADS-box protein of rice, is substituted with threonine (SEQ ID NO: 4). However, the substitution of the amino acid at position 45 in the polypeptide of the present invention is not limited to this. Apart from threonine, the hydrophilic amino acid is glycine, serin, cysteine, aspartic acid, asparagine, glutamic acid, glutamine, lysine, arginine, or histidine. Considering the fact that the superwoman1 (spw1) mutant (see Non-Patent Document 5) has morphologically changed stamens and severe sterility, it cannot be anticipated that cleistogamy is given to a plant by substituting one amino acid of SPW1 by another.

As used herein, the term "polypeptide" can refer to "peptide" or "protein". A "fragment" of the polypeptide means a partial fragment of the polypeptide. A polypeptide of the present invention may be isolated from natural resources or chemically synthesized.

A polypeptide of the present invention may be a native polypeptide taken from natural environment, or a recombinant polypeptide that is expressed in a host cell and is substantially purified by any appropriate technique.

Further, a polypeptide of the present invention may include additional polypeptide. Examples of the additional polypeptide include an epitope-labeled polypeptide which is well-known in the art, such as His, Myc, or Flag.

As used herein in association with a polypeptide or a protein, the term "mutant" means a polypeptide having the activity of giving cleistogamy to a plant. Such a mutant is preferably a polypeptide having deletion, insertion, substitution, or addition of at least one amino acid in an amino acid sequence of the original polypeptide, more preferably a polypeptide having deletion, insertion, substitution, or addition of one or several amino acids in an amino acid sequence of the original polypeptide. Especially, "neutral" amino acid substitution is further preferable since it generally has little effect on the activity of the polypeptide.

In the polypeptide of the present invention, a preferable mutant can have substitution, deletion, or addition of conservative or non-conservative amino acid, which does not change the activity of the polypeptide of the present invention of giving cleistogamy to a plant. It is well known in the art that some amino acids in the amino acid sequence constituting a polypeptide can be easily modified without significant effect on the structure or function of this polypeptide. In addition to the fact that amino acids can be artificially modified, it is also well known that in natural proteins there exists a mutant which does not significantly change the structure or function of the protein. A person skilled in the art can easily mutate one or several amino acids in an amino acid sequence of a polypeptide by using a well-known technique. According to the method described herein, it is possible to easily determine whether a produced mutant has a desired activity.

Thus, a polypeptide of the present embodiment is a polypeptide having the activity of giving cleistogamy to a plant, and a polypeptide comprising an amino acid sequence having substitution of an amino acid at position 45 in the amino acid sequence of a class-B MADS-box protein with a hydrophilic amino acid, or its mutant. In one embodiment, a polypeptide of the present embodiment can comprise (A) an amino acid sequence having substitution of an amino acid at position 45 in an amino acid sequence represented by SEQ ID NO: 1, 6, 8, 10, 12, 14, 16, 18 or 20 with a hydrophilic amino acid; or (B) an amino acid sequence having deletion, insertion, substitution or addition of one or several amino acids other than the amino acid at position 45 in the amino acid sequence (A). In another embodiment, a polypeptide of the present invention can comprise (C) an amino acid sequence having substitution of an amino acid at position 45 in an amino acid sequence represented by SEQ ID NO: 1, 6, or 8 with a hydrophilic amino acid, or (D) an amino acid sequence having deletion, insertion, substitution, or addition of one or several amino acids other than the amino acid at position 45 in the amino acid sequence (C).

### [1-2] Polynucleotide

The present invention also provides a polynucleotide encoding a polypeptide that gives cleistogamy to a plant. In an embodiment, a polynucleotide of the present invention encodes a polypeptide comprising an amino acid sequence having substitution of an amino acid at position 45 in the amino acid sequence of class-B MADS-box protein in a plant with a hydrophilic amino acid, or its mutant.

As used herein, the term "polynucleotide" refers to "gene", "nucleic acid", or "nucleic acid molecule", and means a polymer of nucleotides. As used herein, the term "base sequence" refers to "DNA sequence", "nucleic acid sequence", or "nucleotide sequence".

A polynucleotide of the present invention can exist in the form of DNA (e.g. cDNA or genome DNA) or RNA (e.g. mRNA). The DNA may be single stranded or double stranded. The single strand DNA or RNA may be a coding strand (also known as a sense strand) or a non-coding strand (also known as an anti-sense strand).

In one aspect, a polynucleotide of the present embodiment can be a polynucleotide in which the base at position 348 or 349 in a base sequence represented by SEQ ID NO: 2 is substituted, or its mutant. The base at position 348 or 349 in the base sequence represented by SEQ ID NO: 2 is substituted, whereby the amino acid at position 45 of the encoded polypeptide, a hydrophobic amino acid, is substituted with a hydrophilic amino acid. Preferably, a polynucleotide of the present embodiment can be a polynucleotide having a base sequence represented by SEQ ID NO: 5, or its mutant.

As used herein in association with DNA or polynucleotide, the term "mutant" means a polynucleotide encoding a polypeptide having the activity of giving cleistogamy to a plant. A "polynucleotide mutant having a base sequence represented by SEQ ID NO: 5" means a polynucleotide having deletion, insertion, substitution, or addition of one or several bases in a base sequence represented by SEQ ID NO: 5. Alternatively, the "polynucleotide mutant having a base sequence represented by SEQ ID NO: 5" means a polynucleotide that hybridizes under stringent conditions with a polynucleotide comprising a base sequence complementary to the base sequence represented by SEQ ID NO: 2.

Hybridization can be performed by a well-known method, for example, according to the procedure described in Sambrook et al., Molecular Cloning, A Laboratory Manual, 2d Ed., Cold Spring Harbor Laboratory (1989). As a rule, the level of stringency increases (more difficult to hybridize) with increase in temperature and decrease in salt concentration. Therefore, it is possible to obtain more homologous polynucleotide. An appropriate hybridization temperature varies depending upon a base sequence and its length. For example, in a case where a DNA fragment consisting of 18 bases that encode 6 amino acids is used as a probe, the hybridization temperature is preferably not higher than 50 °C.

As used herein, the "stringent" hybridization conditions mean overnight incubation at 42 °C in a hybridization solution (including 50% formamide, 5 × SSC (150mM NaCl, 15mM trisodium citrate), 50 mM sodium phosphate (pH7.6), 5 x Denhardt's solution, 10% dextran sulfate, and 20 µg/ml of denatured, sheared salmon sperm DNA), followed by washing filters in a 0.1 × SSC at approximately 65 °C. A polynucleotide which hybridizes with a "portion" of polynucleotide means a polynucleotide (either DNA or RNA) that hybridizes with at least approximately 15 nucleotides (nt), more preferably at least approximately 20 nt, still more preferably at least approximately 30 nt, even more preferably longer than approximately 30 nt, of the entire length of the reference polynucleotide. A polynucleotide (oligonucleotide) that hybridizes with such "portion" of polynucleotide is useful as a probe (including primer) discussed in detail herein.

A polynucleotide of the present invention can be fused with a polynucleotide that encodes the foregoing tag label (tag sequence or marker sequence) at its 5'end or 3'end.

Thus, a polynucleotide of the present embodiment is a polynucleotide that encodes a polypeptide having the activity of giving cleistogamy to a plant, and the polynucleotide encodes a polypeptide comprising an amino acid sequence having substitution of an amino acid at position 45 in the amino acid sequence of a class-B MADS-box protein in a plant with a hydrophilic amino acid, or its mutant. In one aspect, a polynucleotide of the present invention is a polynucleotide encoding a polypeptide that gives cleistogamy to a plant, and can be (A) a polynucleotide having a base sequence represented by SEQ ID NO: 5; (B) a polynucleotide having deletion, insertion, substitution, or addition of one or several bases in the base sequence in (A); or (C) a polynucleotide capable of hybridizing with the polynucleotide (A) under stringent conditions. Note that it can be easily understood by a person skilled in the art who reads the specification of the present application that although a polynucleotide of the present invention has been explained taking a base sequence represented by SEQ ID NO: 5 as an example, but a polynucleotide of the present invention is not limited to this.

### [2: Use of polypeptide according to the present invention]

### [2-1] Plant to which cleistogamy is given and method for producing the plant

In one aspect, the present invention provides a plant that expresses a polypeptide of the present invention. In another aspect, the present invention provides a plant that expresses a polynucleotide of the present invention.

In an embodiment, a plant of the present invention can be produced by introducing spw1-cls mutation into a plant that does not have spw1-cls mutation. Note that (i) an amino acid substitution of an amino acid at position 45 of a class-B MADS-box protein with a hydrophilic amino acid and (ii) a base substitution corresponding to the amino acid substitution in a gene that encodes the protein are herein referred to as "spw1-cls mutation" as necessary, and a plant to which cleistogamy is given by these substitutions is referred to as "spw1-cls mutant". Considering the fact that an amino acid near the amino acid at position 45 of the class-B MADS-box protein is mainly constituted by a hydrophobic amino acid, the scope of the present invention should encompass, as the spw1-cls mutant, a mutant obtained by substituting the hydrophobic amino acid other than the amino acid at position 45 with a hydrophilic amino acid.

That is, a method for producing a plant according to the present embodiment comprises the step of expressing a polypeptide of the present invention in a plant. It is preferable that the step of expressing a polypeptide of the present invention in a plant is a step of introducing spw1-cls mutation into a plant. The step of introducing the spw1-cls mutation into a plant can be:
(I) the step of inducing mutation in a gene that encodes the class-B MADS-box protein, wherein the mutation corresponds to an amino acid substitution at position 45 of the class-B MADS-box protein; or
(II) the step of introducing into a plant a gene being capable of inducing mutation in a gene that encodes a class-B MADS-box protein, wherein the mutation corresponds to an amino acid substitution at position 45 of the class-B MADS-box protein.
That is, the step (I) is a step of generating a polynucleotide of the present invention in a plant, and the step (II) is a step of introducing a polynucleotide of the present invention into a plant. The step (I) includes (i) introduction of spw1-cls mutation by gene targeting, or (ii) introduction of spw1-cls mutation by the treatment with a mutagen. However, these are not only possibility. The step (II) includes (iii) introduction of spw1-cls mutant gene or (iv) introduction of spw1-cls mutation by crossing. However, these are not only possibility.
(i) Introduction of spw1-cls mutation by gene targeting
   It is possible to produce a cleistogamous plant by producing a class-B MADS-box protein mutant having deletion, insertion, substitution, or addition of one or several amino acids with respect to an amino acid at a target position or its neighboring position, by using a well-known technique in the art (e.g. gene targeting method (Terada et al., (2002), Efficient gene targeting by homologous recombination in rice. Nat Biotechnol. 20(10): 1030-4)).
(ii) Introduction of spw1-cls mutation by a process using mutagen
   It is possible to produce a cleistogamous plant by processing a target plant with a mutagen so that a class B MADS-box gene locus in the target plant is mutagenized. The inventors of the present invention processed Taichung 65 (*japonica* rice cultivar) with a chemical mutagen N-methyl-N-nitrosourea (MNU) to obtain a mutant population, selected mutant lines in which stamens were not exserted out of glumes at flowering stage from the mutant population, and further selected a line that was normally fertile from the selected lines, so as to obtain a cleistogamous rice mutant, superwomanl-cleistogamy (spw1-cls). Further, mutation of SUPERWOMAN1 (SPW1) gene was confirmed by map-based cloning. It should be noted that a person skilled in the art can select a well-known mutagen in this field appropriately, if necessary.
(iii) Introduction of spw1-cls mutant gene
   As will be described later in Example, it is possible to produce a cleistogamous plant by introducing a genome DNA fragment having spw1-cls mutation into a loss-of-function mutant of a class-B MADS-box gene (e.g. spw1-1 or spw1-2). It should be noted that a person skilled in the art can introduce a gene into a plant successfully and express the gene stably or tissue-specifically, according to a technical knowledge in the art.
(iv) Introduction of spw1-cls mutation by crossing
   It is possible to produce a cleistogamous plant by crossing a cultivar to which cleistogamy is to be given with the spw1-cls mutant produced as described above and selecting a progeny having spw1-cls mutation homozygously from among their progenies.

In another embodiment, a plant of the present invention can be produced by suppressing the expression of a class-B MADS-box gene other than SPW1 in a plant that does not have spw1-cls mutation. Specifically, as will be described later in Example, it is possible to produce a cleistogamous plant by suppressing the expression of a gene that specifically acts on a lodicule (e.g. OsMADS2) among class-B MADS-box genes other than SPW1. In this case, stamens are formed normally by other class-B MADS-box gene that functions redundantly. It should be noted that a person skilled in the art can successfully suppress the expression of a target gene according to a technical knowledge in the art (e.g. knockout method, antisense method, co-suppression method, and RNAi method).

Thus, a person skilled in the art who reads the specification of the present application can easily produce a plant of the present invention by using a well-known technique in the art, and easily select a plant of the present invention by using an oligonucleotide and an antibody, both of which will be described in detail later. In order to screen a target plant (i.e. mutant having spw1-cls mutation), the target plant can be detected by TILLING method (a method for detecting mutation in a target gene locus by specific probe process and mismatched-site specific endonuclease process), etc. Further, it is possible to find out a plant that has amino acid substitution in an amino acid at a target position or its neighboring position or a plant having deletion, insertion, substitution or addition of one or several amino acids, by analyzing a base sequence of SPW1 gene in the obtained mutant.

### [2-2] Oligonucleotide (probe or primer) and use of the same

The present invention provides an oligonucleotide consisting of a fragment of a polynucleotide of the present invention. As used herein, the term "oligonucleotide" refers to a molecule of several to several tens of nucleotides, and it is used interchangeably with "polynucleotide". The oligonucleotide is denoted by the number of nucleotides it contains. For example, the term dinucleotide (dimmer) or trinucleotide (trimer) is used to refer to oligonucleotides of short sequences, whereas long oligonucleotides are referred to as 30-mers or 100-mers. The oligonucleotide may be generated as a fragment of a longer polynucleotide or may be chemically synthesized.

An oligonucleotide of the present invention is useful to select a plant to which cleistogamy is given. That is, by using an oligonucleotide of the present invention, it is possible to easily detect spw1-cls mutation. Therefore, with the present invention, it is possible to easily screen a plant having cleistogamy. Thus, it should be easily understood that the scope of the present invention for screening a plant having cleistogamy encompasses a screening method comprising the step of hybridizing an oligonucleotide of the present invention with a genome DNA or the like derived from a target plant, and a screening kit comprising an oligonucleotide of the present invention.

That is, an oligonucleotide of the present invention includes a fragment of a polynucleotide of the present invention or an oligonucleotide comprising a complementary sequence of the base sequence of the fragment, and may be an oligonucleotide having a base sequence represented by SEQ ID NO: 23. An oligonucleotide set of the present invention includes at least one oligonucleotide described above, and may include an oligonucleotide having a base sequence represented by SEQ ID NO: 22, and an oligonucleotide having a base sequence represented by SEQ ID NO: 23.

Further, a screening method of the present invention is a method for screening a plant to which cleistogamy is given, characterized by comprising the step of hybridizing an oligonucleotide of the present invention with cDNA derived from a target plant, and may comprise the step of hybridizing an oligonucleotide having a base sequence represented by SEQ ID NO: 23 with cDNA derived from a target plant. In this case, it is preferable to further comprises the step of hybridizing an oligonucleotide having a base sequence represented by SEQ ID NO: 22 with cDNA derived from a target plant.

Still further, a screening kit of the present invention is a kit for screening a plant to which cleistogamy is given, the kit comprising an oligonucleotide of the present invention, and may include an oligonucleotide having a base sequence represented by SEQ ID NO: 23. In this case, it is preferable to further comprise an oligonucleotide having a base sequence represented by SEQ ID NO: 22.

Examples of a method for identifying plant variety well known in the art include RFLP method, RAPD method, AFLP method, CAPS method, SSR method, ISSR method, and SNP method. A person skilled in the art who reads the specification of the present application can design a suitable oligonucleotide appropriately according to an adopted method.

It is preferable to use CAPS method (especially dCAPS method), considering that sequence information of a target class-B MADS-box protein has been already known and that method to be used is merely required to detect whether the amino acid substitution at position 45 of the class-B MADS-box protein occurs. The CAPS method is a method for cutting a PCR product with a particular restriction enzyme to detect a base substitution (polymorphism) according to variations in length of the obtained fragments. In a case where there is no preferable restriction enzyme site, it is possible to newly provide a preferable restriction site by devising a primer (oligonucleotide) (dCAPS method).

For example, a first oligonucleotide (5'-TGCAGAACTCGTGGTACTTGCCGGTGGAGGAGAACACCATG-3' (SEQ ID NO: 23)) has a partial base substitution introduced in a template for amplification (5'-TGCAGAACTCGTGGTACTTGCCGGTGGAGGAGAACATAATG-3 '), that is a complementary sequence of SPW1 DNA (SEQ ID NO: 2) or of SPW1 genome DNA (SEQ ID NO: 3). In order to forma pair of primers with the first oligonucleotide and be capable of amplifying a fragment containing a spw1-cls mutation site, a second oligonucleotide (e.g. 5'-CAGGGAGCTCACCGTGCTCTGCGACGCCCAGGTCGCCA-3' (SEQ ID NO: 22)) is designed. In a case where fragments amplified by PCR using the first and second oligonucleotides as a pair of primers are NcoI-processed, the amplified fragment is cut at a site (CCATGG) generated when there is spw1-cls mutation. On the other hand, the amplified fragment is not cut at a site (TCATGG) generated when there is no spw1-cls mutation.

The above descriptions have explained the first oligonucleotide that is a dCAMPS marker, taking as an example an oligonucleotide having a base sequence represented by SEQ ID NO: 23. However, note that an oligonucleotide of the present invention is not limited to this. Further, the above descriptions have explained the second oligonucleotide that forms a pair of primers, taking as an example an oligonucleotide having a base sequence represented by SEQ ID NO: 22. However, an oligonucleotide of the present invention is not limited to this.

Applicant of the present invention has developed a novel technique of identifying single base polymorphism (see Japanese Unexamined Patent Publication No. 2004-248635 (published on September 9, 2004). In an embodiment, an oligonucleotide of the present invention can be an oligonucleotide to be applied to the above technique. That is, an oligonucleotide of the present embodiment is a PCR primer for identifying a genotype of single base polymorphism, the first base from 3'end of the primer corresponds to the position of the single base polymorphism to be identified, and the third base from 3'end of the primer is substituted with a base other than one complementary to a template sequence annealed with the primer. The above substitution is preferably G to T substitution, A to C substitution, T to G substitution, or C to A substitution. A Tm value of an oligonucleotide of the present embodiment is not less than 50 °C, preferably not less than 60 °C, more preferably not less than 65 °C, most preferably 70 °C. By using an oligonucleotide of the present embodiment, it is possible to know existence of single base polymorphism (single base substitution) in a target gene from whether a target amplification product can be obtained.

As used herein, the term "primer" means a single strand oligonucleotide that acts as an origin of template-directed DNA synthesis under appropriate conditions. A preferable length of the primer, which can be changed appropriately according to an intended use, can be generally a length corresponding to nucleotides with any number of bases in the range from 15 to 50 nucleotides, preferably 15 to 30 nucleotides. A sequence of the primer does not need to match a template sequence completely, but the sequence of the primer should be sufficiently complementary to the template sequence in order to hybridize the primer with the template. The term "a pair of primers" means a primer set including (i) 5' (upstream) primer which hybridizes with 5'-end of a nucleic acid sequence to be amplified and (ii) 3' (downstream) primer which hybridizes with a complement of 3'-end of the nucleic acid sequence to be amplified.

The "PCR (polymerase chain reaction)" is described herein as an exemplary nucleic-acid amplification reaction. However, an oligonucleotide of the present embodiment can be applied to a nucleic-acid amplification reaction other than PCR.

That is, an oligonucleotide of the present invention is characterized by being a fragment of an polynucleotide of the present invention or an oligonucleotide comprising a complementary sequence of the base sequence of the fragment, and applicable to a variety identification technique well known in the art. Therefore, a person skilled in the art who reads the specification of the present application easily understands that an oligonucleotide of the present invention is designed appropriately according to each technique adopted to detect spw1-cls mutation and is not limited to a particular oligonucleotide.

### [2-3] Antibody and use of the same

The present invention provides an antibody being capable of binding specifically to a polypeptide of the present invention. An antibody of the present invention is not limited if it can bind specifically to a polypeptide of the present invention, and may be a polyclonal antibody or the like against the polypeptide. However, the antibody of the present invention is preferably a monoclonal antibody against the polypeptide of the present invention. The monoclonal antibody has the advantages of having uniform properties, being easily supplied, and being semipermanently preserved as a hybridoma, and other advantages.

As used herein, the term "antibody" means immunoglobulin (IgA, IgD, IgE, IgG, IgM, and their Fab fragments, F(ab')₂ fragments, Fc fragments). Examples of the antibody include a polyclonal antibody, a monoclonal antibody, a single strand antibody, and an antiidiotype antibody. However, the antibody of the present invention is not limited to these antibodies.

As used herein, the wording "bind specifically to a polypeptide of the present invention" means being capable of binding specifically to a polypeptide in which the amino acid at position 45 of the class-B MADS-box protein is substituted with a hydrophilic amino acid, but not bind to a polypeptide in which the amino acid at position 45 of the class-B MADS-box protein is not substituted. Therefore, by using the present invention, it is possible to easily screen a plant having cleistogamy. Thus, it should be easily understood that the scope of the present invention for screening a plant having cleistogamy encompasses a screening method comprising the step of incubating an antibody of the present invention with a tissue extract derived from a target plant, and a screening kit comprising an antibody of the present invention.

An antibody of the present invention is characterized by being capable of binding specifically to a polypeptide of the present invention. A screening method of the present invention is a method for screening a plant to which cleistogamy is given, and characterized by comprising the step of incubating the above antibody with a tissue extract derived from a target plant. Further, a screening kit of the present invention is a kit for screening a plant to which cleistogamy is given and
characterized by comprising the above antibody.

As used herein, the wording "antibody being capable of binding specifically to a polypeptide of the present invention" means that the antibody includes complete antibody molecules and antibody fragments (e.g. Fab fragment and F(ab')₂ fragment) both of which can bind specifically to the polypeptide of the present invention. The Fab fragment and F(ab')₂ fragment lack Fc portion of a complete antibody, the Fc portion is removed more speedily through circulation, and the Fab fragment and F(ab')₂ fragment hardly have a character of non-specific tissue binding of a complete antibody (Wahl et al., J. Nucl. Med. 24: 316-325 (1983) (herein incorporated by reference)) Therefore, these fragments are preferable.

The "antibody" can be produced according to various publicly known methods (e.g. HarLow et al., Antibodies: A laboratory manual, Cold Spring Harbor Laboratory, New York (1988), and Iwasaki et al., Monoclonal antibody hybridoma and ELISA, Kodansha (1991)).

The monoclonal antibody can be produced by a well-known method in the art (for example, see hybridoma method (Kohler, G. and Milstein, C., Nature 256, 495-497 (1975)), trioma technique and human-B cell hybridoma technique (Kozbor, Immunology Today 4,72 (1983)) and EBV-hybridoma method (Monoclonal Antibodies and Cancer Therapy, Alan R Liss, Inc., 77-96 (1985))).

The peptide antibody can be also produced by a well-known method in the art (for example, see Chow, M. et al., Proc. Natl. Acad. Sci. USA 82: 910-914; and Bittle, F.J. et al., J. Gen. Virol. 66: 2347-2354 (1985)).

It is clear for a person skilled in the art that Fab fragments, F(ab')₂ fragments, and other fragments of an antibody of the present invention can be used according to a method disclosed herein. These fragments can be produced through cutting by proteolysis using a typical enzyme such as papain (which generates Fab fragments) or pepsin (which generates F(ab')₂ fragments). Alternatively, a fragment being capable of binding to a polypeptide of the present invention can be produced by use of recombinant DNA technique or chemical synthesis.

Thus, an antibody of the present embodiment has only to include a fragment being capable of binding specifically to a polypeptide of the present invention (e.g. Fab fragment and F(ab')₂ fragment), and it should be noted that the present invention encompasses an immunoglobulin composed of (i) the fragment being capable of binding specifically to a polypeptide of the present invention and (ii) Fc fragment of another antibody.

That is, an object of the present invention is to provide an antibody being capable of binding specifically to a polypeptide of the present invention and use of the same, without limitations to types of individual immunoglobulins specifically described herein (IgA, IgD, IgE, IgG, or IgM), peptide antigen producing method, and others. Therefore, it should be noted that the scope of the present invention encompasses an antibody obtained by a method other than the foregoing methods.

Further, all of the academic documents and patent documents listed herein are incorporated by reference herein.

### [Example]

### [1: Methods]

### [1-1. Identification of cleistogamous mutant]

To find cleistogamous mutants, M2 population of rice (*Oryza sativa* L. subsp. *japonica* cv. Taichung 65) mutagenized with N-methyl-N-nitrosourea (MNU) were screened. The M2 population was cultivated under usual conditions. Wild-type rice flowers were opened at flowering stage and closed after 15 minutes. Some anthers remained outside the glumes even after the glumes were closed. Thus, first, plants in which anthers were not observed outside the glumes at flowering stage were selected. Subsequent examination reveled one fertile line in which the cleistogamy was inherited as a single recessive trait. To confirm stability of cleistogamy of this mutant in different genetic background, this mutant was crossed with cv. Kasalath(subsp. *indica*)*,* and the segregation of cleitogamy in the F2 population were examined.

### [1-2. Paraffin sectioning]

Flower buds of wild type and spw1-cls were fixed in FAA (formaldehyde : glacial acetic acid : ethanol = 1:1:18) for 24 hours at 4 °C, and then dehydrated in a graded ethanol series. Dehydrated samples in 100 % ethanol were replaced with xylene, and embedded in Paraplast plus (Oxford Labware, St. Louis, MO). Microtome sections at 8 µm thick were stained with Delafiled's hematoxyline and observed with a light microscope.

### [1-3. Scanning electron microscopy (SEM)]

Fresh samples were observed directly through a scanning electron microscopy (VE-8000, Keyence, Osaka, Japan).

### [1-4. In situ hybridization]

Paraffin sections were prepared as described above except that microtome sections at 8 mm thick were applied to slide glasses coated with APS (Matsunami Glasses, Osaka, Japan). Digoxigenin-labeled antisense and sense probes were prepared from the full-length or partial cDNAs of SPW1,OsMADS2 and OsMADS4. In *situ* hybridiation and immunological detection of the hybridization signals were performed as described in Kouchi, H. and Hata, S. Mol.Gen.Genet.238, 106-119 (1993).

### [1-5. Genetic Mapping]

For map-based cloning, F2 population of the homozygous cleistogamous plant and cv.Kasalath (subsp. *indica*) was used. Using CAPS markers, the causal gene was mapped on the long arm of chromosome 6.

### [1-6. Plant transformation]

All the genomic DNA fragments or cDNAs (with or without mutations) were amplified by PCR reaction using PfuUltra DNA polymerase (Stratagene) and were verified by sequencing. The DNA fragments were cloned into pZH2B (gifted from Mr. M. Kuroda). For RNAi experiments, 312bp (SPW1), 300bp (OsMADS2) and 58bp (OsMADS4) of gene-specific DNA fragments were cloned in head-to-head orientation into pZH2Bi (gifted from Mr. M. Kuroda) with the rice polyubiquitin gene promoter and the third intron of rice aspartic protease gene. Vectors were introduced into rice scutellum-derived calli by Agrobacterium-mediated transformation under selection by Hygromycin according to a method described in Oikawa et al., Plant Mol. Biol. 55, 687-700 (2004). spw1-cls or spw1-1 homozygous calli were selected with allele-specific dCAPS markers (SEQ ID NO: 22 and 23).

### [1-7. Yeast two-hybrid assay]

Coding sequences corresponding to M domain, I domain and K domain of the wild type or mutant (I45T or V45T) of SPW1, OsMADS2 and OsMADS4 were amplified using PfuUltra DNA polymerase (Stratagene), verified by sequencing and subcloned into the two-hybrid vectors, pAS2-1 and pACT2. All the yeast transformation and (β-galactosidase liquid assays were performed as described in Wang, K.L. et al., Nature 428, 945-950 (2004), except that the yeast cells were incubated at 20°C.

### [2: Phenotypes of spw1-cls having cleistogamy]

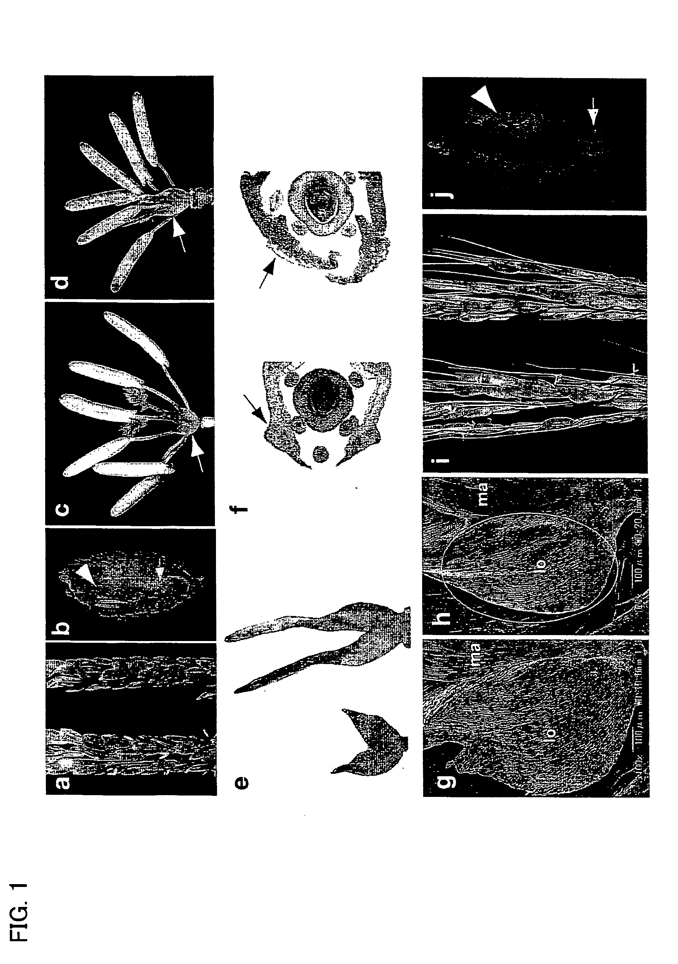

In Fig. 1, (a) shows appearance of a segregated line exhibiting a wild-type trait (left), and spw1-cls (right), for comparison in cleistogamy therebetween. In Fig. 1, (b) shows development of a rice grain of spw1-cls in which part of glume is cut. An arrow indicates immature seed, and arrowhead indicates stamens. As is apparent from this, stamens were remained in a glume in spw1-cls.

In Fig. 1, (c) and (d) show structures of internal floral organs of wild type and spw1-cls mutant. A lodicule (indicated by an arrow) of a wild type was not elongated (c), but a lodicule (indicated by an arrow) of spw1-cls was elongated although other floral organs were normal (d). (e) shows comparison in shape of lodicules between wild type (left) and spw1-cls (right). In cleistogamous rice spw1-cls, a lodicule was transformed to be elongated, but other organs such as stamens were not changed.

(f) shows transverse sections of wild type (left) and spw1-cls (right) florets. Arrows indicates lodicules. A lodicule of the wild type was enlarged. A lodicule of wild-type rice had many vascular bundles developed, while a lodicule of the spw1-cls mutant had immature vascular bundles and a flat structure. (g) and (h) show scanning electron micrographs of lodicules (lo) and marginal regions of paleae (ma) of the wild type and the spw1-cls mutant. In the wild-type rice, the lodicule had a surface composed of round and swollen cells (g). In spw1-cls, the lodicule had a surface composed of elongated and flat cells observed in the marginal regions of paleae (h). From these results, it is considered that spw1-cls could not flower since an organ to be developed as a lodicule was transformed to a lodicule-glume mosaic organ, and as a result, cells of the organ lost the swelling capability.

In Fig. 1, (i) shows F2 individual expressing wild type (chasmogamy) obtained by crossing indica rice cultivar, Kasalath, with spw1-cls mutant, and F2 individual expressing spw1-cls (cleistogamy) on the right side. From F2 population obtained by crossing of spw1-cls with indica rice cultivar "Kasalath", 25 % of cleistogamous individuals were obtained. It was found that the cleistogamy trait was expressed stably independent of genetic backgrounds, i.e. cleistogamy was expressed stably even with the change in genetic backgrounds.

In Fig. 1, (j) shows a post-fertilization flower of the spw1-cls mutant into which 35S promoter:: GFP gene was introduced as a foreign gene. In (j), part of a glume is removed so that the inside of the glume can be observed. It is apparent that green fluorescence caused by high expression of the GFP gene and cleistogamy were observed, and ovary was developed with stamens remaining inside the caryopsis. Thus, it was confirmed that cleistogamy was expressed stably even when spw1-cls mutant was used as a mother plant for genetic recombination.

The d7 mutant known as "Heieidaikoku" is cleistogamous rice reported so far. In the d7 mutant, the proximal parts of lemma and palea are fused tightly while having normal lodicules, resulting in the non-opened flowers at flowering stage. The d7 mutant, however, is presumed to cause other growth disturbances (e.g. dwarf grain and short grain) due to pleiotropic expressions of causal genes, thus is not suitable for practical use.

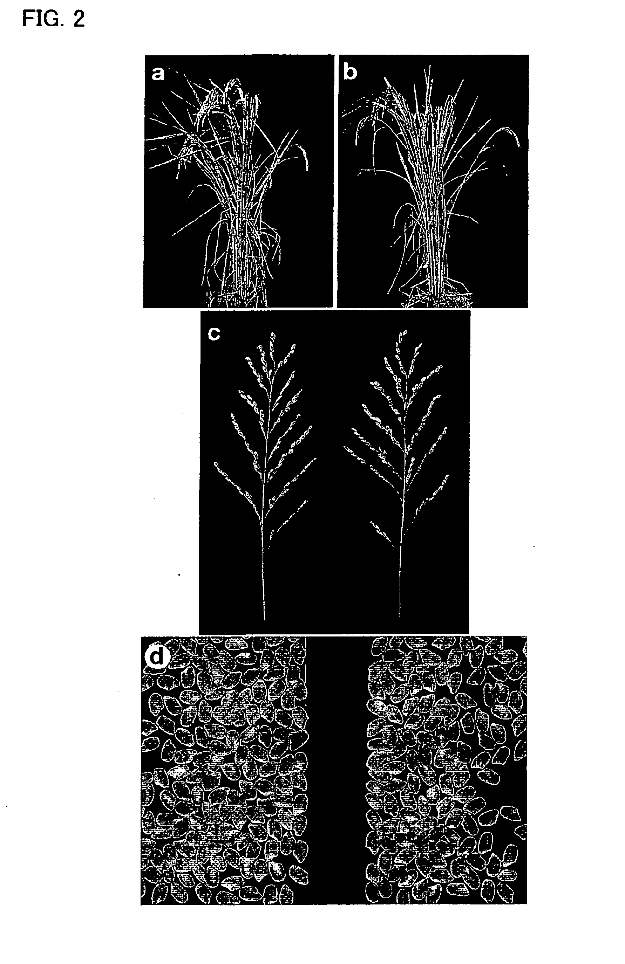

In view of this, plant shape and panicle shape were compared between spw1-cls mutant having cleistogamy and a wild type (Fig. 2). (a) shows plant shape of a segregant expressing wild-type traits, and (b) shows plant shape of spw1-cls. (c) shows comparison between panicles of wild type (left) and those of spw1-cls (right). (d) shows comparison in rice grain appearance between wild type (left) and spw1-cls mutant (right). It is apparent that the appearance of spw1-cls is similar to that of wild type, except that spw1-cls has cleistogamy. In addition, spw1-cls has seed fertility rate equal to that of wild-type rice, panicles are exserted normally, and there was no significant differences in other traits (plant type, heading date, etc.) between spwl-cls and wild-type rice (Table 1).

**[Table 1]**

| | | | | | | | |
|---|---|---|---|---|---|---|---|
| Agronomic traits of spw1-cls | | | | | | | |

| | Heading date | Maximal plant height | No. of panicles | Maximal panicle length | No. of grains | Fertility | Grain weight |
|---|---|---|---|---|---|---|---|
| | (days) | (cm) | (per plant) | (cm) | (per panicle) | (%) | (mg) |
| Wild type | 116.1±2.5 | 92.30±2.56 | 14.6±1.9 | 22.64±1.26 | 113.1±14.8 | 87.55±5.69 | 20.99±0.65 |
| Spw1-cls | 116.8±2.0 | 90.53±3.53 | 13.0±2.4 | 23.70±1.11 | 127.9±16.7 | 72.97±11.33 | 19.58±0.82 |

As described above, spw1-cls is considered to have influence specifically on the properties of lodicules, and spw1-cls mutant is suitable for practical use.

### [3: Mechanism of cleistogamy in spw1-cls]

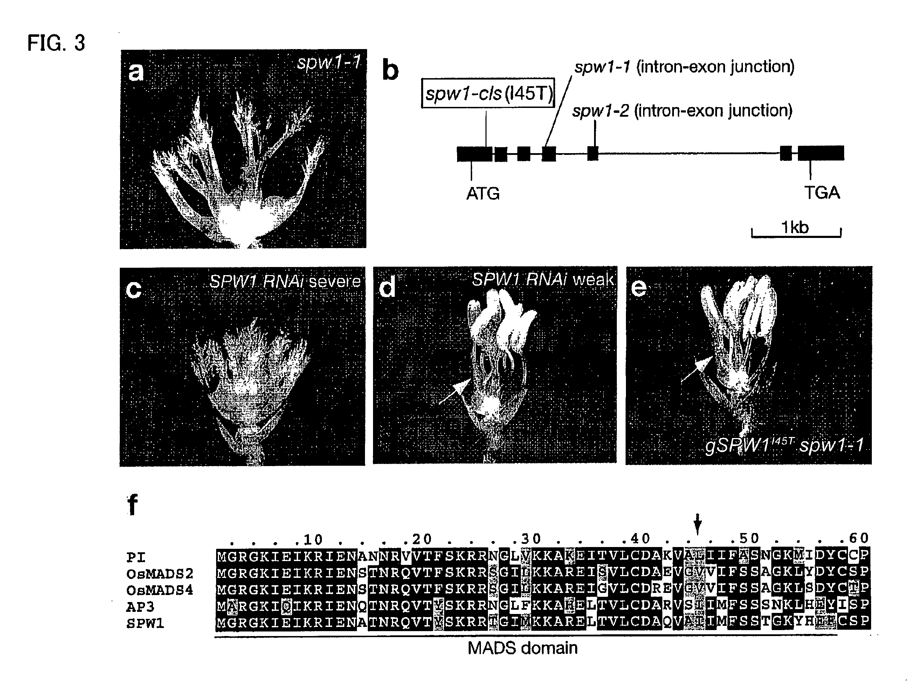

In order to reveal mechanism in which cleistogamy is brought by mutation of SPW1 gene, flowers of SPW1-gene-related transformed plants were analyzed (Fig. 3). Fig. 3 shows (a) the structures of floral organs of a loss-of-function mutant spw1-1 of SPW1 gene, (c) the structures of floral organs of a line in which expression of SPW1 gene was severely suppressed by RNAi method, and (d) the structures of floral organs of a line in which expression of SPW1 gene was mildly suppressed by RNAi method.

As shown in Fig. 3, when the amount of expression of SPW1 gene was reduced by RNAi method, the followings were observed: (a) spw1-1 and spw1-2 (not shown) that are allele of loss-of-function mutation of SPW1, (c) the structures having an elongated lodicule and pistil-like stamens, and (d) the structures having an elongated lodicule as in spw1-cls, but normal stamen appearance.
In a case where the expression of SPW1 gene was suppressed severely, an elongated lodicule and pistil-like stamens were observed as in the case of the loss-of-function mutant spw1-1. In a case where the expression of SPW1 gene was suppressed mildly, only an elongated lodicule was observed as in the spw1-cls mutant. However, these lines had transformed pollens and were completely sterile.

In Fig. 3, (e) shows function complementation of a loss-of-function mutant spw1-1 by a mutated SPW1 gene. (e) of Fig. 3 shows that introduction of the mutated SPW1 gene having I45T mutation (gSPW1^{I45T}) into spw1-1 results in spw1-cls type flower having an elongated lodicule and normal stamens. As a result of this, it was found that appropriately weakening the function of the SPW1 gene induced morphological change similar to that of spw1-cls, and also it was found difficult to secure pollen fertility and seed fertility merely by reducing the amount of expression of SPW gene.

In the present invention, it was verified by map-based cloning that cleistogamy was induced by mutation of SUPERWOMAN1 (SPW1) gene. SPW1 is classified as class-B of MADS-box protein transcriptional activator, and class-B MADS-box proteins are further classified into PISTILLATA (PI) sub-family and APETALLA3 (AP3) sub-family. Further, proteins that belong to the subfamilies form heterodimer, and the heterodimer binds to DNA having a particular sequence called CArG box so that transcription of genes is activated. In "ABC model" for formation of floral organs, which model has been proposed for *Arabidopsis* and other plants, it is known that in dicotyledons, the class-B MADS-box proteins are required for formation of their petals and stamens, and in mocotyledons, the class-B MADS-box proteins are required for formation of their lodicules and stamens. It is considered that SPW 1, which is the sole AP3-type class-B MADS-box protein of rice, forms a heterodimer with PI-type proteins OsMADS2 and OsMADS4, and thus controls the formation of lodicules and stamens. In Fig. 3, (b) is a view showing the structure of SPW1 gene. Boxes represent exons, thin lines represent introns, ATG indicates translation initiation codon, and TGA indicates translation stop codon. spw1-1and spw1-2 bring loss-of-function mutation (superwoman mutation) due to splicing defect, resulting in transformation of lodicules to glume-like organs and transformation of stamens to pistils. In spw1-cls mutant, the amino acid at position 45 (isoleucine(I)) is substituted with threonine (T).

In Fig. 3, (f) shows comparison between class-B MADS-box proteins (*Arabidopsis* (PI, AP3) and rice (SPW1, OsMADS2, OsMADS4)) in amino acid sequences of MADS domains. In (f) of Fig. 3, reversed characters indicate that amino acids are conserved in three or more proteins out of the five proteins. Shaded areas indicate that amino acids having similar properties are conserved. In wild-type class-B MADS-box proteins, all of the amino acids at position 45 (indicated by an arrow) are hydrophobic amino acids (leucine (L), valine (V), isoleucine (I)), which are highly conserved. However, in spw1-cls, the amino acid at position 45 is substituted with a hydrophilic amino acid, threonine (T), suggesting that the structure of the spw1-cls protein is changed slightly from the structure of the wild-type proteins. Considering that an amino acid near the amino acid at position 45 of the class-B MADS-box protein is mainly a hydrophobic amino acid, the scope of the present invention should encompass that spw1-cls mutant is obtained by substituting the hydrophobic amino acid other than the amino acid at position 45 with a hydrophilic amino acid.

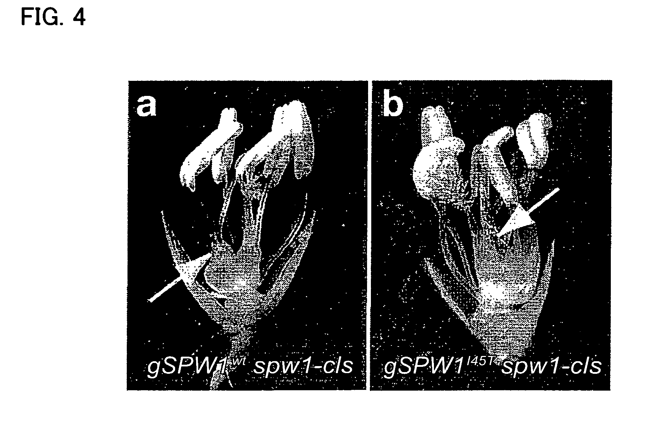

Next, it was confirmed that the spw1-cls mutation was caused by amino acid substitution (mutation of the 45th isoleucine to threonine (I45T)) in MADS-box domain (DNA-binding protein domain) (Fig. 4). (a) of Fig. 4 shows function complementation of spw1-cls mutant by a wild-type SPW1 gene. Introduction of wild-type SPW1 gene (gSPW1^{wt}) into a spw1-cls mutant resulted in a wild-type flower (in which lodicule (indicated by an arrow) was not elongated). On the other hand, even when a mutant SPW1 gene (gSPW1^{I45T}) was introduced into a spw1-cls mutant, spw1-cls flower having a lodicule remained elongated and having normal stamens was obtained (b). From these results, it was found that I45T mutation was the cause of spw1-cls mutation. dCAPS markers (SEQ ID NO: 22 and 23) produced by using this mutation enables cleistogamy-determination on young plants. With the use of the DNA markers, it is possible to successfully select and effectively breed cultivars into which clestogamy is introduced by crossing.

Flowering of many *Poaceae* plants such as rice is considered to occur due to the swelling of lodicules. According to the present invention, similar mutation can be given to a class-B MADS-box gene so as to properly decrease the heterodimer-forming ability, whereby it is possible to produce or select cleistogamous plants in many *Poaceae* plants.

### [4: Interaction of class-B MADS-box proteins]

As described previously, SPW1 identified as a causal gene of cleistogamy is one of "class-B MADS-box genes" known as genes controlling the formation of morphology of petals (lodicules) and stamens in a wide range of higher plants. It is known that class-B MADS-box proteins, products of these genes, are classified into two sub-class proteins, AP3-type proteins and PI-type proteins, and heterodimer formed with a AP3-type subclass protein and a PI-type subclass protein binds to DNA to activate the transcription of gene cluster required for the formation of morphology of organs. SPW1 is the sole AP3-type protein in rice and considered to form heterodimer with PI-type proteins, OsMADS2 and OsMADS4. In view of this, it was analyzed by yeast two-hybrid method using I45T SPW1 proteins (SPW1^{I45T}) whether formations of heterodimers to be formed between SPW1 proteins and two PI-type proteins can be effected by spw1-cls mutation.

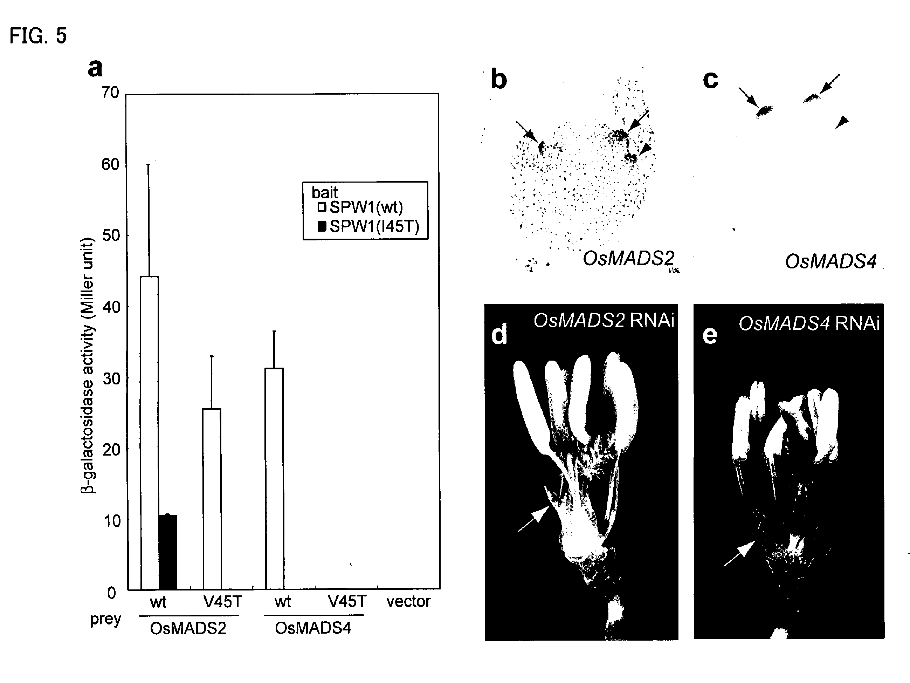

In Fig. 5, (a) shows the result by yeast two-hybrid method, analyzing protein-to-protein interaction between wild-type or I45T mutant-type SPW1 protein and OsMADS2 protein or OsMADS4 protein. By using intensity of a reporter gene (LacZ gene) expression as an index was measured the interaction between wild-type SPW1 protein (SPW1(wt): white column) or mutant-type SPW1 protein (SPW1(I45T): black column) and wild-type protein or V45T mutant-type protein of OsMADS2, and the interaction between wild-type SPW1 protein (SPW1(wt)) or mutant-type SPW1 protein (SPW1 (I45T)) and wild-type protein or V45T mutant-type protein of OsMADS4. As a result, it was found that the heterodimer-forming ability of the I45T SPW1 protein (SPW1^{I45T}) decreased. In (a), the more tightly two proteins are bound to each other, the higher values are. It is clear that wild-type SPW1^{wt} is bound to wild-type OsMADS2 and OsMADS4 while mutant-type SPW1^{I45T} is little bound to OsMADS4, and the degree of binding of mutant-type SPW1^{I45T} with OsMADS2 is in the range from 1/2 to 1/10 the degree of binding of wild-type SPW1^{wt} with OsMADS2. Further, V45T mutants of OsMADS2 and OsMADS4 were produced to confirm the binding of the V45T mutants of OsMADS2 and OsMADS4 with SPW1^{wt}. As a result, it was found that binding of OsMADS2V45T with SPW1^{wt} was about half binding of OsMADS2wt with SPW1^{wt}, and OsMADS4V45T is little bound to SPW1^{wt}. In addition, it was found that OsMADS2V45T and OsMADS4V45T were not bound to SPW1^{I45T} at all.

In Fig. 5, (b) and (c) show expression sites of OsMADS2 gene and OsMADS4 gene using *in situ* hybridization. As a result of analysis by *in situ* hybridization, it was found that OsMADS2 expressed in primordia of lodicule and stamen (b), and OsMADS4 expressed only in primordia of stamen (c). These results indicate that in the spw1-cls mutant, in lodicule primordia, the formation of lodicule was inhibited due to weak binding of SPW1^{I45T} with OsMADS2, and in stamen primordia, while SPW1^{I45T} is not bound to OsMADS4 at all, a sufficient activity for the formation of stamens is maintained although binding of SPW1^{I45T} to OsMADS2 is weak.

Since OsMADS4 does not express in lodicule primordia, the interaction between SPW and OsMADS2 is considered to be important for the formation of lodicules. This is supported by the fact that a low level of heterodimer formation between SPW1^{I45T} and OsMADS2 is sufficient to form stamens, but insufficient to establish complete properties of lodicules. To verify this idea, expressions of OsMADS2 gene and OsMADS4 gene were suppressed by RNAi method. In Fig. 5, (d) and (e) show the structures of floral organs in lines in which the expressions of the OsMADS2 gene and the OsMADS4 gene were suppressed by RNAi method.

In the case of a plant in which the expression of the OsMADS2 gene was suppressed, normal stamens and elongated glume-like lodicule were exhibited ((d) in Fig. 5) since OsMADS4 mRNA did not exist in the second whorl, but existed in the third whorl. On the contrary, in the case of a plant in which the expression of the OsMADS4 gene was suppressed, phenotypes of stamens and lodicules were not exhibited since OsMADS2 existed in both the second whorl and the third whorl.

From the above results, it is considered that in spw1-cls, the heterodimer-forming ability of the SPW1 protein, which is a class-B MADS-box protein, with OsMADS2 or OsMADS decreased, and as a result, class-B activity decreased, so that morphological change was caused only in lodicules, but not in stamens.

With the present invention, it is possible to easily produce a plant to which cleistogamy is given. Further, with the present invention, it is possible to easily screen plants having cleistogamy.

Specific embodiments or examples implemented in BEST MODE FOR CARRYING OUT THE INVENTION only show technical features of the present invention and are not intended to limit the scope of the invention. Variations can be effected within the spirit of the present invention and the scope of the following claims.

### INDUSTRIAL APPLICABILITY

Cultivars having chasmogamy release their pollens and easily receive pollens of other varieties. Therefore, there have been concerns about crossing and/or gene flow of the cultivars. Self-pollination is impossible without retaining of the function of stamens. The present invention provides cleistogamous cultivars that have been desired, and makes a significant contribution to efficiency of breeding.

Conventionally, in order to determine whether a plant has cleistogamy, it was necessary to observe flowering states of individual plants and visually check whether anthers were out of glume at flowering stage. In addition, it was difficult to select individuals having cleistogamy and retaining the function of stamens. The present invention provides a genetic marker by which a plant having cleistogamy can be successfully selected and makes contribution to considerably efficient plant breeding.

Thus, the present invention has various applications in the agricultural field, and can also be effectively used in the food industry. In addition, the present invention can be used in a basic research in the biological field.

## Claims

1. A polypeptide which gives cleistogamy to a plant, wherein the polypeptide is: (A) a polypeptide comprising an amino acid sequence having substitution of an amino acid at position 45 in the amino acid sequence of a class-B MADS-box protein with a hydrophilic amino acid; or (B) a polypeptide comprising an amino acid sequence having deletion, insertion, substitution, or addition of one or several amino acids other than the amino acid at position 45 in the amino acid sequence of the polypeptide (A).

2. The polypeptide according to claim 1, wherein the hydrophilic amino acid is threonine.

3. A polynucleotide encoding the polypeptide according to claim 1.

4. A plant expressing the polypeptide according to claim 1.

5. A plant expressing the polynucleotide according to claim 3.

6. A method for producing a plant to which cleistogamy is given, comprising any one of:
(I) the step of inducing mutation in a gene that encodes a class-B MADS-box protein, wherein the mutation corresponds to an amino acid substitution at position 45 of the class-B MADS-box protein;
(II) the step of introducing into a plant a gene being capable of inducing mutation in a gene that encodes a class-B MADS-box protein, wherein the mutation corresponds to an amino acid substitution at position 45 of the class-B MADS-box protein; and
(III) the step of suppressing expression of genes encoding class-B MADS-box protein other than a polypeptide having an amino acid sequence represented by SEQ ID NO: 1.

7. A kit for producing a plant to which cleistogamy is given, wherein the kit comprises the polynucleotide according to claim 3.

8. A fragment of the polynucleotide according to claim 3, or an oligonucleotide comprising a complementary sequence of the base sequence of the fragment.

9. A method for screening a plant to which cleistogamy is given, comprising the step of hybridizing the oligonucleotide according to claim 8 with cDNA derived from a target plant.

10. A kit for screening a plant to which cleistogamy is given, wherein the kit comprises the oligonucleotide according to claim 8.

11. An antibody being capable of binding specifically to the polypeptide according to claim 1.

12. A method for screening a plant to which cleistogamy is given, comprising the step of incubating the antibody according to claim 11 with a tissue extract derived from a target plant.

13. A kit for screening a plant to which cleistogamy is given, wherein the kit comprises the antibody according to claim 11.
